# FASCICULE DE BREVET EUROPEEN

(11) **EP 1 899 298 B1**
(45) Date de publication et mention de la délivrance du brevet: **18.08.2010**
(21) Numéro de dépôt: 06764809.7
(22) Date de dépôt: 22.06.2006
(51) Int. Cl.: C07D 207/32, A61K 31/40

(54) **DERIVES DE 4,5-DIARYLPYRROLE, LEUR PREPARATION ET LEUR APPLICATION EN THERAPEUTIQUE**
DERIVATE VON 4,5-DIARYLPYRROL, VERFAHREN ZU DEREN HERSTELLUNG UND DEREN VERWENDUNG IN THERAPEUTIKA
DERIVATIVES OF 4,5-DIARYLPYRROLE, PREPARATION METHOD THEREOF AND USE OF SAME IN THERAPEUTICS

(30) Priorité: 27.06.2005 FR 0506609
(43) Date de publication de la demande: 19.03.2008
(73) Titulaire: Sanofi-Aventis, 75013 Paris (FR)
(72) Inventeur: BARTH, Francis, F-34680 Saint Georges d'Orques (FR); CONGY, Christian, F-34980 Saint Gely du Fesc (FR); HORTALA, Laurent, F-34080 Montpellier (FR); RINALDI-CARMONA, Murielle, F-34680 Saint Georges d'Orques (FR)
(74) Mandataire: Kugel, Dominique
(86) Numéro de dépôt international: PCT/FR2006/001416
(87) Numéro de publication internationale: WO 2007/000505

(56) Documents cités:
- WO-A-2004/058249
- US-A- 4 335 136
- US-A- 5 935 990
- LANGE J H M ET AL: "Keynote review: Medicinal chemistry strategies to CB1 cannabinoid receptor antagonists" DRUG DISCOVERY TODAY, ELSEVIER, RAHWAY, NJ, US, vol. 10, no. 10, 15 mai 2005 (2005-05-15), pages 693-702, XP004894759 ISSN: 1359-6446

## Description

La présente invention a pour objet des dérivés de 4,5-diarylpyrrole, leur préparation et leur application en thérapeutique.

Le brevet américain US 4 335 136 et le brevet européen EP 0 038 536 décrivent des dérivés de 4,5-diaryl-alpha(polyfluoralkyl)-1*H*-pyrrole-2-méthanamine présentant des propriétés antiinflammatoires.

La demande de brevet WO 2005/058 249 décrit des dérivés de diarylpyrrole en tant que modulateurs des récepteurs aux cannabinoïdes et la demande de brevet WO 2003/027 069 décrit des dérivés de diarylpyrrole actifs sur l'obésité.

On a maintenant trouvé des nouveaux dérivés de 4,5-diaryl-2-aminométhylpyrrole qui possèdent des propriétés antagonistes des récepteurs CB₁ des cannabinoïdes, localisés au niveau central et / ou périphérique.

La présente invention a pour objet des composés répondant à la formule (I): 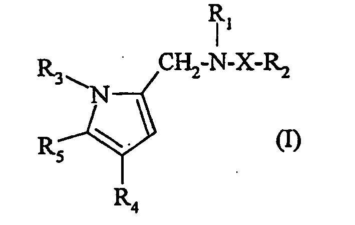 dans laquelle :
- X représente un groupe 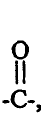 -SO₂-, 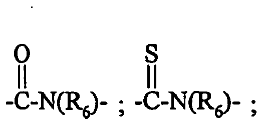
- R₁ représente un atome d'hydrogène ou un groupe (C₁-C₄)alkyle ;
- R₂ représente :
   - un (C₁-C₁₂)alkyle non substitué ou substitué une ou plusieurs fois par des substituants choisis indépendamment parmi un atome d'halogène, un hydroxyle, un (C₁-C₄) alcoxy, un (C₁-C₄)alkylthio, un phénoxy, un radical trifluorométhoxy, un radical difluorométhoxy, un radical difluorométhylthio, un radical trifluorométhylthio ;
   - un radical carbocyclique non aromatique en (C₃-C₁₂) non substitué ou substitué une ou plusieurs fois par des substituants choisis indépendamment parmi un (C₁-C₄)alkyle, un (C₁-C₄)alcoxy, un (C₁-C₄)alkylthio, un atome de fluor, un hydroxyle, un radical trifluorométhyle, difluorométhyle, trifluorométhoxy, difluorométhoxy, trifluorométhylthio, difluorométhylthio ;
   - un méthyle substitué par un radical carbocyclique non aromatique en (C₃-C₁₂) non substitué ou substitué une ou plusieurs fois par des substituants choisis indépendamment parmi un (C₁-C₄)alkyle, un (C₁-C₄)alcoxy, un (C₁-C₄)alkylthio, un atome de fluor, un hydroxyle, un radical trifluorométhyle, difluorométhyle, trifluorométhoxy, difluorométhoxy, trifluorométhylthio, difluorométhylthio ;
   - un radical phényle, benzyle, benzhydryle, benzydrylméthyle, dans lequel chaque groupe phényle est non substitué ou substitué une ou plusieurs fois par des substituants choisis indépendamment parmi un atome d'halogène, un hydroxyle, un (C₁-C₄)alkyle, un (C₁-C4)alcoxy, un méthylènedioxy, un cyano, un nitro, un trifluorométhyle, un difluorométhyle, un difluorométhoxy, un trifluorométhoxy, un trifluorométhylthio, un difluorométhylthio, un groupe S(O)ₙAlk, un groupe OS(O)ₙAlk, un groupe (C₁-C₄)alkylcarbonyle ;
   - un radical phényle substitué par un radical hétérocyclique choisi parmi pyrrolyle, imidazolyle, pyridyle ou pyrrazolyle, ledit radical hétérocyclique étant non substitué ou substitué une ou plusieurs fois par un ou plusieurs substituants choisis indépendamment parmi un atome d'halogène ou un groupe (C₁-C₄)alkyle ;
   - un radical phényle substitué par un phényle ou un phénoxy dans lequel chaque groupe phényle est non substitué ou substitué une ou plusieurs fois par des substituants choisis indépendamment parmi un atome d'halogène, un hydroxyle, un (C₁-C₄)alkyle, un (C₁-C₄)alcoxy, un méthylènedioxy, un cyano, un nitro, un trifluorométhyle, un difluorométhyle, un difluorométhoxy, un trifluorométhoxy, un trifluorométhylthio, un difluorométhylthio, un groupe S(O)ₙAlk, un groupe OS(O)ₙAlk, un groupe (C₁-C₄)alkylcarbonyle ;
   - un 1,2,3,4-tétrahydronaphtalèn-2-yle non substitué ou substitué une ou plusieurs fois par un (C₁-C₄)alkyle ou un trifluorométhyle ;
   - un radical pyrrolyle, imidazolyle, pyridyle, pyrazolyle, furyle, thiényle, oxazolyle, thiazolyle, lesdits radicaux étant non substitués ou substitués par un ou plusieurs substituants choisis indépendamment parmi un atome d'halogène, un groupe (C₁-C₄)alkyle ou trifluorométhyle ;
   - un indol-2-yle ou un N-méthylindol-2-yle ;
- R₃ représente un (C₁-C₅)alkyle ou un (C₃-C₇) cycloalkyle ;
- R₄ représente un phényle non substitué ou substitué une ou plusieurs fois par des substituants choisis indépendamment parmi un atome d'halogène, un (C₁-C₄)alkyle, un (C₁-C₄)alcoxy, un cyano, un radical difluorométhyle, trifluorométhyle, difluorométhoxy, trifluorométhoxy, un groupe S(O)ₙAlk ou OS(O)ₙAlk ;
- R₅ représente un phényle non substitué ou substitué une ou plusieurs fois par des substituants choisis indépendamment parmi un atome d'halogène, un (C₁-C₄)alkyle, un (C₁-C₄)alcoxy, un cyano, un radical difluorométhyle, trifluorométhyle, difluorométhoxy, trifluorométhoxy, un groupe S(O)ₙAlk ou OS(O)ₙAlk ;
- R₆ représente un atome d'hydrogène ou un (C₁-C₄)alkyle ;
- n représente 0, 1 ou 2 ;
- Alk représente un (C₁-C₄)alkyle.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères ainsi que leurs mélanges, y compris les mélanges racémiques, font partie de l'invention.

Les composés de formule (I) peuvent exister sous forme d'hydrates ou de solvats, à savoir sous forme d'associations ou de combinaisons avec une ou plusieurs molécules d'eau ou avec un solvant. De tels hydrates et solvats font également partie de l'invention.

Par atome d'halogène on entend un atome de brome, de chlore, de fluor ou d'iode. Par (C₁-C₄)alkyle ou respectivement (C₁-C₅)alkyle ou (C₁-C₇)alkyle, on entend un radical alkyle linéaire ou ramifié de un à quatre atomes de carbone ou respectivement de un à cinq atomes de carbone ou de un à sept atomes de carbone, tel que le radical méthyle, éthyle, propyle, isopropyle, butyle, isobutyle, *sec-*butyle, *tert-*butyle, pentyle, isopentyle, hexyle, isohexyle, heptyle.

Par (C₁-C₄)alcoxy on entend un radical alcoxy linéaire ou ramifié de un à quatre atomes de carbone tel que le radical méthoxy, éthoxy, propoxy, isopropoxy, butoxy, *sec-*butoxy, *tert*-butoxy.

Par un radical carbocyclique non aromatique en C₃-C₁₂ on entend : un radical monocyclique ou un radical di- ou tricyclique condensé ou ponté ; par radical monocyclique on entend un cycloalkyle, par exemple cyclopropyle, cyclobutyle, cyclopentyle, cyclohexyle, cycloheptyle, les radicaux cyclopentyle, cyclohexyle et cycloheptyle étant préférés ; par radical di- ou tricyclique condensé ou ponté, on entend par exemple le bicyclo[2.2.1]heptyle, le bicyclo[2.2.2]octyle, le bicyclo[3.2.1]octyle, l'adamantyle.

Tout particulièrement, la présente invention a pour objet des composés de formule (I) dans laquelle :
- X représente un groupe 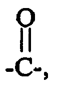 -SO₂-, 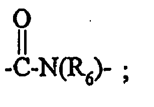
- R₁ représente un atome d'hydrogène ou un groupe (C₁-C₄)alkyle ;
- R₂ représente :
   - un (C₁-C₇)alkyle ;
   - un radical carbocyclique non aromatique en C₃-C₁₂ non substitué ou substitué une ou plusieurs fois par un groupe (C₁-C₄)alkyle ;
   - un méthyle substitué par un radical carbocyclique non aromatique en C₃-C₁₂ et non substitué ou substitué une ou plusieurs fois sur le carbocycle par un (C₁-C₄)alkyle ;
   - un phényle non substitué ou substitué une ou plusieurs fois par des substituants choisis indépendamment parmi un atome d'halogène, un (C₁-C₄)alkyle, un hydroxyle, un (C₁-C₄)alcoxy, un cyano, un groupe trifluorométhyle, un groupe trifluorométhoxy, un groupe S(O)ₙAlk, un groupe (C₁-C₄)alkylcarbonyle ; ou parmi un radical phényle, phénoxy, pyrrolyle, imidazolyle, pyridyle ou pyrazolyle, lesdits radicaux étant non substitués ou substitués une ou plusieurs fois par un (C₁-C₄)alkyle ;
   - un benzyle non substitué ou substitué une ou plusieurs fois sur le phényle par des substituants choisis indépendamment parmi un atome d'halogène, un cyano, un (C₁-C₄)alkyle, un (C₁-C₄)alcoxy, un radical trifluorométhyle ou un groupe S(O)ₙAlk ; ou substitué en alpha par un ou deux groupes semblables ou différents choisis parmi un (C₁-C₄)alkyle et un (C₃-C₇)cycloalkyle ;
   - un benzhydryle, un groupe benzhydrylméthyle ;
   - un 1,2,3,4-tétrahydronaphtalèn-2-yle non substitué ou substitué une ou plusieurs fois par un (C₁-C₄)alkyle ;
   - un radical pyrrolyle, imidazolyle, pyridyle, pyrazolyle, furyle, thiényle, lesdits radicaux étant non substitués ou substitués par un ou plusieurs substituants choisis indépendamment parmi un atome d'halogène ou un groupe (C₁-C₄)alkyle ;
   - un indol-2-yle ou un N-méthylindol-2-yle ;
- R₃ représente un (C₁-C₅)akyle ou un (C₃-C₇) cycloalkyle ;
- R₄ représente un phényle non substitué ou substitué une ou plusieurs fois par des substituants choisis indépendamment parmi un atome d'halogène, un (C₁-C₄)alkyle, un (C₁-C₄)alcoxy, un radical trifluorométhyle ou un groupe S(O)ₙAlk ;
- R₅ représente un phényle non substitué ou substitué une ou plusieurs fois par des substituants choisis indépendamment parmi un atome d'halogène, un (C₁-C₄)alkyle, un (C₁-C₄)alcoxy, un radical trifluorométhyle ou un groupe S(O)ₙAlk ;
- R₆ représente un atome d'hydrogène ou un (C₁-C₄)alkyle ;
- n représente 0, 1 ou 2 ;
- Alk représente un (C₁-C₄)alkyle.

Parmi les composés de formule (I), objets de l'invention, on distingue :
- les composés de formule (IA) dans laquelle -X- représente un groupe -CO- et les substituants R₁ à R₅ sont tels que définis pour les composés de formule (I) ;
- les composés de formule (IB) dans laquelle -X- représente un groupe -SO₂- et les substituants R₁ à R₅ sont tels que définis pour les composés de formule (I) ;
- les composés de formule (IC) dans laquelle -X- représente un groupe -CON(R₆)- et les substituants R₁ à R₆ sont tels que définis pour les composés de formule (I) ;
- les composés de formule (ID) dans laquelle -X- représente un groupe -CSN(R6)-et les substituants R₁ à R₆ sont tels que définis pour les composés de formule (I).

Parmi les composés objets de l'invention, on préfère les composés de formule (I) dans laquelle :
- R₁ représente un atome d'hydrogène ;
- R₂ a l'une des valeurs définies pour (I) ;
- R₃ représente un groupe méthyle ;
- R₄ et R₅ représentent un 2,4-dichlorophényle et un 4-chlorophényle, un 2,4-dichlorophényle et un 4-bromophényle, un 2-chlorophényle et un 4-chlorophényle, un 2,4-dichlorophényle et un 4-méthoxyphényle ;
- X représente un groupe -CO- ; -SO₂- ou -CON(R₆)- ;
ainsi que leurs hydrates ou leurs solvats.

On préfère tout particulièrement les composés de formule (I) dans laquelle les substituants R₁, R₃, R₄, R₅ et X sont tels que définis ci-dessus et R₂ représente un groupe choisi parmi :
- un 3-chlorophényle, un 3-trifluorométhylphényle, un 4-trifluorométhylphényle, un hept-4-yle ;
ainsi que leurs hydrates ou leurs solvats.

Conformément à l'invention, on peut préparer les composés de formule (I) selon un procédé qui est caractérisé en ce que :
on traite un composé de formule : 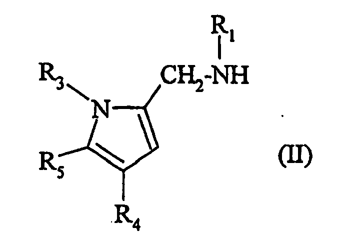 dans laquelle R₁, R₃, R₄ et R₅ sont tels que définis pour un composé de formule (I) :
   - soit par un acide ou un dérivé fonctionnel de cet acide de formule :

      HOOC-R₂ (III)
   dans laquelle R₂ est tel que défini pour un composé de formule (I), lorsqu'on doit préparer un composé de formule (I) dans laquelle -X- représente un groupe -CO- ;
   - soit par un halogénure de sulfonyle de formule :

      Hal-SO₂-R₂ (IV)
   dans laquelle R₂ est tel que défini pour un composé de formule (I) et Hal représente un atome d'halogène, préférentiellement le chlore, lorsqu'on doit préparer un composé de formule (IB) dans laquelle -X- représente un groupe -SO₂- ;
   - soit par un halogénoformiate de formule :

      HalCOOAr (V)
   dans laquelle Hal représente un atome d'halogène et Ar représente un phényle ou un 4-nitrophényle pour obtenir un composé intermédiaire de formule : 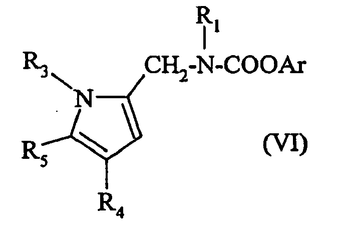 dans laquelle R₁, R₃, R₄ et R₅ sont tels que définis pour un composé de formule (I), que l'on fait réagir ensuite avec une amine de formule :

   HNC(R₆)R₂ (VII)

   dans laquelle R₂ et R₆ sont tels que définis pour un composé de formule (I), lorsqu'on doit préparer un composé de formule (IC) dans laquelle -X- représente un groupe - CON(R₆)- ;
   - soit par un isothiocyanate de formule R₂-N=C=S (IX) dans laquelle R₂ est tel que défini pour un composé de formule (I) lorsque l'on doit préparer un composé de formule I(D) dans laquelle -X- représente un groupe -CSN(R₆)-.

Eventuellement, on transforme le composé de formule (I) en l'un de ses sels d'addition à un acide.

Lorsqu'on traite un composé de formule (II) avec l'acide de formule (III) lui-même, on opère en présence d'un agent de couplage utilisé en chimie peptidique tel que le 1,3-dicyclohexylcarbodiimide (DCC) ou l'hexafluorophosphate de benzotriazol-1-yloxytris(diméthylamino) phosphonium (BOP) ou l'hexafluorophosphate de benzotriazol-1-yloxytris(pyrrolidino) phosphonium (PyBOP) ou le tétrafluoroborate de 2-(1*H-*benzotriazol-1-yl)-1,1,3,3-tétmméthyl uronium (TBTU), en présence d'une base telle que la triéthylamine, la N,N-diisopropyléthylamine ou la 4-diméthylaminopyridine, dans un solvant tel que le dichlorométhane, le dichloroéthane, le N-N-diméthylformamide ou le tétrahydrofurane à une température comprise entre - 10°C et la température de reflux du solvant.

Comme dérivé fonctionnel de l'acide (III) on peut utiliser le chlorure d'acide, l'anhydride, un anhydride mixte, un ester alkylique en C₁-C₄ dans lequel l'alkyle est droit ou ramifié, un ester activé, par exemple l'ester de p-nitrophényle.

Ainsi dans le procédé selon l'invention, on peut aussi faire réagir le chlorure de l'acide obtenu par réaction du chlorure de thionyle ou du chlorure d'oxalyle sur l'acide de formule (III), avec le composé de formule (II), dans un solvant, tel qu'un solvant chloré (le dichlorométhane, le dichloroéthane, le chloroforme par exemple), un éther (tétrahydrofurane, dioxane par exemple), ou un amide (N,N-diméthylformamide par exemple) sous une atmosphère inerte, à une température comprise entre 0°C et la température ambiante, en présence d'une amine tertiaire telle que la triéthylamine, la N-méthylmorpholine ou la pyridine.

Une variante consiste à préparer l'anhydride mixte de l'acide de formule (III) par réaction du chloroformiate d'éthyle avec l'acide de formule (III), en présence d'une base telle que la triéthylamine, et à le faire réagir avec le composé de formule (II), dans un solvant tel que le dichlorométhane, sous une atmosphère inerte, à la température ambiante, en présence d'une base telle que la triéthylamine.

Lorsqu'on traite un composé de formule (II) avec un halogénure de sulfonyle de formule (IV), on opère en présence d'une base telle que la triéthylamine ou la diisopropyléthylamine, dans un solvant tel que le dichlorométhane ou le tétrahydrofurane et à une température comprise entre la température ambiante et la température de reflux du solvant.

Lorsqu'on traite un composé de formule (II) avec un halogénoformiate de formule (V), on opère en présence d'une base telle que la triéthylamine, dans un solvant tel que le dichlorométhane et à une température comprise entre 0°C et la température ambiante. Puis on fait réagir le composé intermédiaire de formule (VI) ainsi obtenu avec une amine de formule (VII), dans un solvant tel que le dichlorométhane, en présence d'une base telle que la triéthylamine et à une température comprise entre 0°C et la température de reflux du solvant.

Selon une variante du procédé on peut préparer les composés de formule (IC) dans laquelle -X- représente un groupe -CON(R₆)- dans lequel R₆ = H par réaction d'un composé de formule (II) avec un isocyanate de formule R₂-N=C=O (VIII), en présence d'une base telle que la triéthylamine, dans un solvant tel que le dichlorométhane et à une température comprise entre la température ambiante et la température de reflux du solvant.

Selon une autre variante du procédé on peut préparer les composés de formule (IC) dans laquelle -X- représente un groupe -CON(R₆)- par réaction d'un composé de formule (II) avec un composé de formule ClCON(R₆)R₂ (IX) en présence d'une base telle que la triéthylamine, dans un solvant tel que le dichlorométhane et à une température comprise entre 0°C et la température ambiante.

Les composés de formule (I) dans laquelle R₁ représente un (C₁-C₃)alkyle peuvent aussi être préparés à partir des composés correspondant de formule (I) dans laquelle R₁ représente un atome d'hydrogène par une méthode choisie parmi les méthodes connues de l'homme de l'art. Parmi celles-ci on peut citer l'alkylation par un halogénure d'alkyle, l'animation réductrice par un aldéhyde en milieu réducteur, ou encore l'acylation par un chlorure d'acyle, suivi d'une réduction.

Les composés de formule (I) ainsi obtenus peuvent être ultérieurement séparés du milieu réactionnel et purifiés selon les méthodes classiques, par exemple par cristallisation ou chromatographie.

Les composé de formule (II) peuvent être préparés selon le schéma réactionnel suivant :

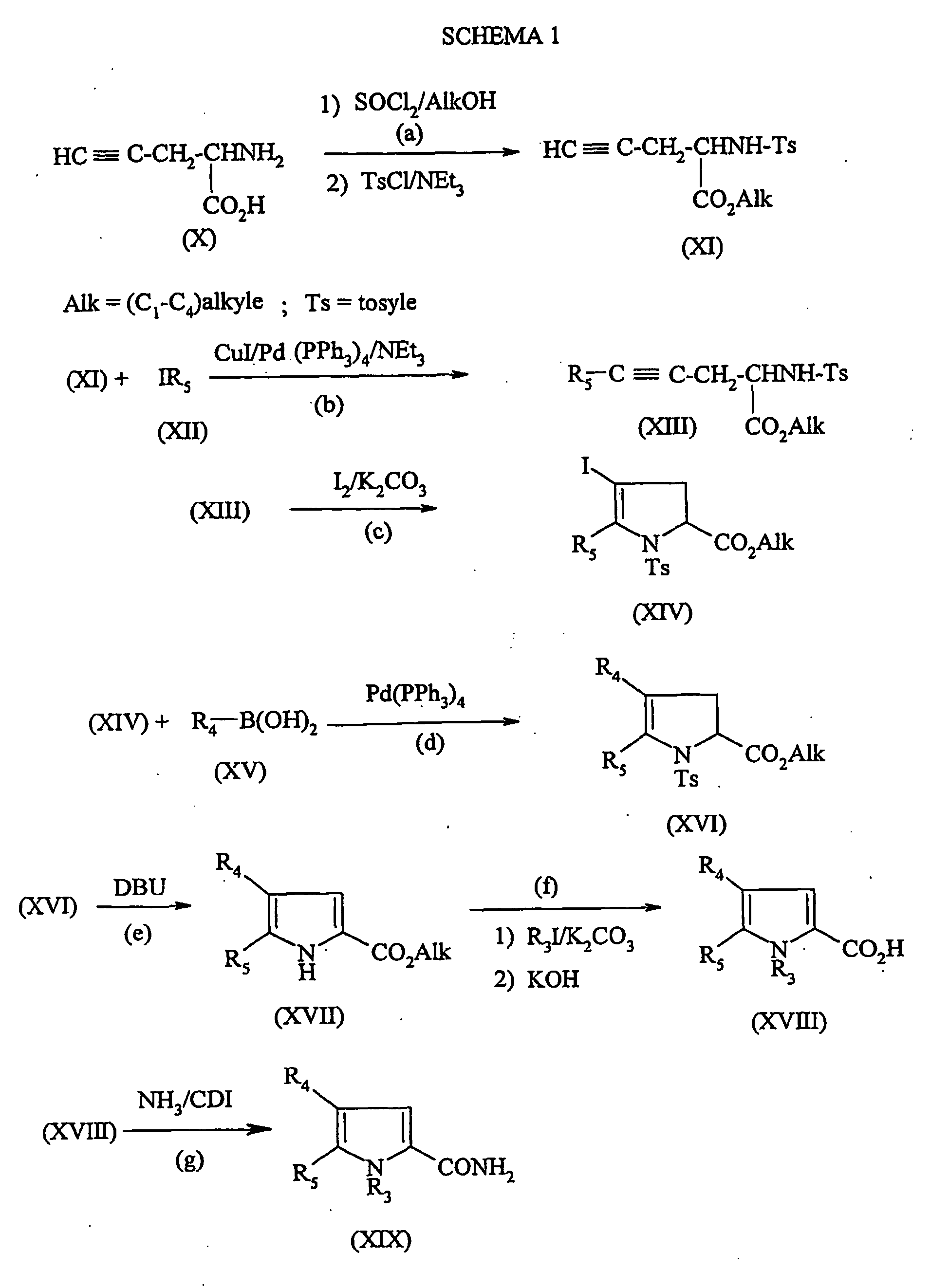

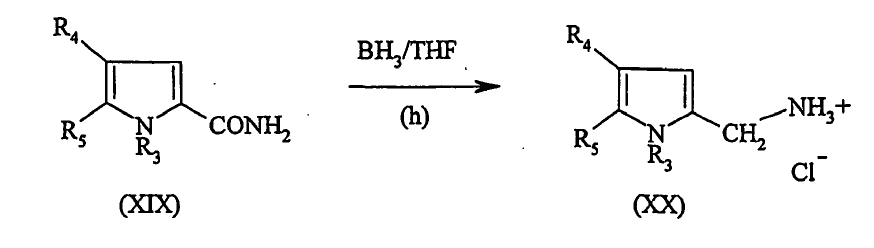

La préparation du dérivé de dihydropyrrole de formule (XIV)
par les étapes a), b) et c) est effectuée selon J. Chem. Soc. Perkin Trans. 1, 2002, 622-628.

Les composés de formule (III) sont connus.

Les composés de formule (IV) sont disponibles dans le commerce ou décrits dans la littérature, ou peuvent être préparés selon des méthodes qui y sont décrites telles que dans J. Org. Chem. USSR, 1970, 6, 2454-2458 ; J. Am. Chem. Soc., 1952, 74, 2008 ; J. Med.Chem., 1977, 20(10), 1235-1239 ; EP0469 984 ; WO95/18105.

Par exemple les composés de formule (IV) peuvent être préparés par halogénation des acides sulfoniques correspondants ou de leurs sels, par exemple de leur sels de sodium ou de potassium. La réaction s'effectue en présence d'un agent halogénant tel que l'oxychlorure de phosphore, le chlorure de thionyle, le trichlorure de phosphore, le tribromure de phosphore ou le pentachlorure de phosphore, sans solvant ou dans un solvant tel qu'un hydrocarbure halogéné ou le N,N-diméthylformamide et à une température comprise entre -10°C et 200°C.

La substitution du noyau dihydropyrrole par un groupe phényle substitué (R₄) est effectuée à l'étape d) par action d'un acide phénylboronique substitué de formule (XV) en présence d'un catalyseur au palladium tel que le tetrakis(triphénylphosphine)[Pd(PPh₃)₄], le palladium (0)bisdibenzylidène acétone [Pd(dba)₂], le tris(dibenzylideneacétone)dipalladium(0), l'acétate de palladium Pd(II)[Pd(OCOCH₃)₂], le dichloro(diphénylphosphinoferrocène) Pd(II) [PdCl₂dppf], et en présence d'une base.

A l'étape e), la protection de l'azote par le groupe tosyle est enlevée par action d'une diamine telle que DBU(1,8-diazabicyclo[5.4.0]undécène) simultanément le noyau pyrrole est aromatisé.

A l'étape f), on alkyle l'azote du pyrrole par action d'un iodure d'alkyle de formule R₃I, puis on hydrolyse l'ester en milieu basique pour obtenir, après acidification, l'acide de formule (XVIII).

A l'étape (g), l'acide de formule (XVIII) est traité par de l'ammoniac gazeux dans un solvant polaire aprotique, tel que le DMF ou l'acétonitrile, en présence d'un agent de couplage tel que le CDI (1,1'-carbonyl bis(1*H-*imidazole)), ou le 1,3-dicyclohexylcarbodiimide (DCC) ou l'hexafluorophosphate de benzotriazol-1-yloxytris(diméthylamino) phosphonium (BOP) ou l'hexafluorophosphate de benzotriazol-1-yloxytris(pyrrolidino) phosphonium (PyBOP).

A l'étape (h), la réduction de la fonction carboxamide du composé de formule (XIX) est effectuée au moyen d'un agent réducteur tel que le borane ou l'hydrure de lithium et d'aluminium, dans un solvant tel que le tétrahydrofurane ou l'éther isopropylique, à une température comprise entre la température ambiante et la température de reflux du solvant, suivi d'une hydrolyse acide.

Les composés de formule (II) sont nouveaux. Ainsi la présente invention a également pour objet les composés de formule : 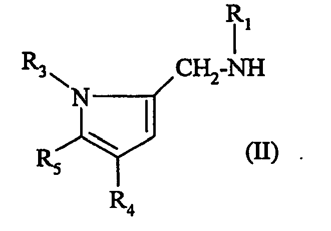 dans laquelle R₁, R₃, R₄ et R₅ sont tels que définis pour (I).

Plus particulièrement, la présente invention a pour objet les composés de formule (II) dans laquelle :
- R₁=H;
- R₃=Me;
- R₄ = 2,4-dichlorophényle ou 2-chlorophényle ;
- R₅ = 4-chlorophényle, 4-bromophényle ou 4-méthoxyphényle.

Les EXEMPLES suivants décrivent la préparation de certains composés conformes à l'invention. Ces exemples ne sont pas limitatifs et ne font qu'illustrer la présente invention. Les numéros des composés exemplifiés renvoient à ceux donnés dans le TABLEAU I ci-après, qui illustre les structures chimiques et les propriétés physiques de quelques composés selon l'invention.

Dans les Préparations et dans les Exemples on utilise les abréviations suivantes :
éther : éther diéthylique
éther iso : éther diisopropylique
DMSO : diméthylsulfoxyde
DMF : N,N-diméthylformamide
THF : tétrahydrofurane
TBTU : tétrafluoroborate de 2-(1*H-*benzotriazol-1-yl)-1,1,3,3-tétraméthyluronium
DCM : dichlorométhane
AcOEt : acétate d'éthyle
DIPEA : diisopropyléthylamine
BH₃-THF : complexe de borane-tetrahydrofurane
DBU : 1,8-diazabicyclo[5,4,0]undec-7-ène
TFA : acide trifluoroacétique
Ether chlorhydrique 2N : solution 2N d'acide chlorhydrique dans l'éther diéthylique
F : point de fusion
TA : température ambiante
Eb : température d'ébullition
CLHP : chromatographie liquide haute performance
Silice H : gel de silice 60 H commercialisé par Merck (DARMSTAD)
Solution tampon pH = 2 : solution de 16,66 g de KHSO₄ et 32,32 g de K₂SO₄ dans 1 litre d'eau.

Les spectres de résonance magnétique nucléaire du proton (RMN ¹H) sont enregistrés à 200 MHz dans le DMSO-d₆. Les déplacements chimiques δ sont exprimés en parties par million (ppm). Pour l'interprétation des spectres, on utilise les abréviations suivantes : s : singulet, d : doublet, t : triplet, q : quadruplet, m : massif, mt : multiplet, se : singulet élargi, dd : doublet dédoublé.

Les composés selon l'invention sont analysés par couplage LC/UV/MS (chromatographie liquide/détection UV/spectrométrie de masse). On mesure le pic moléculaire (MH⁺) et le temps de rétention (tr) en minutes.

### Conditions A :

On utilise une colonne Symmetry C18 de 2,1 x 50 mm, 3,5 µm, à 30°C, débit 0,4 ml/minute.

L'éluant est composé comme suit :
- solvant A : 0,005 % d'acide trifluoroacétique (TFA) dans l'eau à pH 3,15 ;
- solvant B : 0,005 % de TFA dans l'acétonitrile.

### Gradient :

| Temps (mn) | % A | % B |
|---|---|---|
| 0 | 100 | 0 |
| 10 | 10 | 90 |
| 15 | 10 | 90 |
| 16 | 100 | 0 |
| 20 | 100 | 0 |

La détection UV est effectuée à λ = 210 nm et la détection de masse en mode ionisation chimique ESI (de l'anglais Electro Spray Ionisation) positif.

### Conditions MS2

On utilise une colonne XTERRA MS C18 de 2,1 x 30 mm, 3,5 µm, débit 0,8 ml/minute.

L'éluant est composé comme suit :
Solvant A : 0,025 % de TFA dans l'eau,
Solvant B : 0,025 % de TFA dans l'acétonitrile.

### Gradient

| Temps (mn) | % A | % B |
|---|---|---|
| 0 | 100 | 0 |
| 2 | 0 | 100 |
| 2,7 | 0 | 100 |
| 2,75 | 100 | 0 |

La détection UV est effectuée par un détecteur à barette d'iode entre 210 et 400 nm et la détection de masse en mode ESI positif.

### PREPARATIONS

### Préparation 1

### A) 2-(((4-méthylphényl)sulfonyl)amino)pent-3-ynoate de méthyle.

2,5 g d'acide 2-aminobut-3-ynoique sont mis en suspension dans 45 ml de méthanol à 0°C. On coule au goutte à goutte 1,8 ml de chlorure de thionyle à cette température puis le mélange est chauffé à reflux pendant 3 heures. La solution est concentrée et le résidu est séché sous pression réduite. Ce dernier est solubilisé dans 60 ml d'acétonitrile suivi de 5,4 ml de triéthylamine puis on ajoute 4,6 g de chlorure de tosyle. Le mélange est agité à température ambiante pendant 19 heures puis à 50°C une heure supplémentaire. Après concentration, le brut est solubilisé dans le dichlorométhane et la phase organique est lavée successivement par une solution aqueuse saturée de KHSO₄ puis de K₂CO₃. La phase organique est séchée sur sulfate de magnésium puis filtrée et enfin concentrée pour obtenir 5,18 g du composé attendu.

RMN¹H : δ (ppm) : 2,35 s : 3H ; 2,45 : m : 2H ; 3,45 : s : 3H ; 3,9 : dd : 1H ; 7,35 : d : 2H ; 7,65 : d : 2H ; 8,4 : d : 1H.

### B) 5-(4-chlorophényl)-2-(4-tosylamino)pent-4-ynoate de méthyle.

1 g du composé de l'étape précédente et 0,57 g de 4-chloroiodobenzène sont solubilisés dans 20 ml de DMF anhydre. La solution est dégazée sous vide pendant 30 minutes. On ajoute 0,64 ml de triéthylamine puis 0,28 g de tetrakis(triphénylphosphinepalladium(0) et 0,1g de iodure de cuivre. Le mélange est agité à température ambiante sous atmosphère d'argon pendant 19 heures. Le mélange réactionnel est concentré et purifié par chromatographie sur gel de silice cyclohexane/acétate d'éthyle (80/20 ; v/v). On récupère 1 g du composé attendu.

RMN¹H : δ (ppm) : 2,35 : s : 3H ; 2,70-2,80 : m : 2H ; 3,45 : s : 3H ; 4,05 : dd : 1H ; 7,35 : m : 4H ; 7,4 : d : 2H ; 7,65 : d : 2H ; 8,51 : d : 1H.

### C) 5-(4-chlorophényl-4-iodo-1-(4-tosylsulfonyl)-2,3-dihydro-1H-pyrrole-2-carboxylate de méthyle.

On dissout 1 g du composé obtenu à l'étape précédente dans 5 ml d'acétonitrile anhydre en présence de 1g de carbonate de potassium à 0°C. Sous agitation à cette température, on ajoute 2 g d'iode solide en plusieurs petites fractions. Le mélange est laissé revenir à température ambiante pendant 24 heures. La réaction est arrêtée par addition d'une solution de thiosulfate de sodium jusqu'à décoloration et la phase organique est extraite au dichlorométhane. Après séchage sur sulfate de magnésium, filtration et concentration, on obtient 1,27g du composé attendu.

LC/MS : M = 517, tr = 10,8 minutes.

### D) 5-(4-chlorophényl)-4-(2,4-dichlorophényl)-1-tosylsulfonyl-2,3-dihydro-1H-pyrrole-2-carboxylate de méthyle

15 g du composé obtenu à l'étape précédente et 6,8 g de l'acide 2,4-dichlorophényle boronique sont solubilisés dans un mélange de 150 ml de méthanol, 710 ml de toluène, en présence de 48 ml d'une solution de carbonate de sodium (2N). On laisse le milieu réactionnel sous argon pendant 30 minutes puis on ajoute 4,7g de tetrakis(triphénylphoshine)palladium(0). La solution est chauffée à 60°C pendant 4 heures sous atmosphère inerte. Après refroidissement, le brut est concentré et purifié par chromatographie sur gel de silice dans le toluène. On obtient 9,7 g du composé attendu sous forme d'une poudre blanche.

RMN¹H : δ (ppm) : 2,4 : s : 3H ; 2,75-2,95 : m : 1H ; 3,8 : s : 3H ; 5,15 : d : 1H ; 6,7 : d : 1H ; 7,1-7,7 : m : 6H.

### E) 5-(4-chlorophényl)-4-(2,4-dichlorophényl)-1H-pyrrole-2-carboxylate de méthyle.

9,7 g du composé obtenu à l'étape précédente sont solubilisés dans 60 ml de N,N-diméthylformamide anhydre. Puis 5,4 ml de DBU sont ajoutés et le mélange est chauffé à 100°C pendant 24 heures. Le brut est concentré puis après ajout d'éthanol, un précipité blanc apparaît. Ce dernier est filtré, on recueille 6 g du composé attendu.

RMN¹H : δ (ppm) : 3,8 : s : 3H ; 6,9 : s : 1H ; 7,2 : s : 1H ; 7,25 : s : 2H ; 7,3-7,4 : m:3H;7,65:dd: 1H; 12,4 : s : 1H.

### F) Acide 5-(4-chlorophényl)-4-(2,4-dichlorophényl)-1-méthyl-1H-pyrrole-2-carboxylique.

5,9 g du composé obtenu à l'étape précédente sont solubilisés dans 150 ml de DMF et 3,5 g de carbonate de potassium sont ajoutés. A température ambiante, 1,5 ml de iodométhane sont ajoutés au mélange et on laisse sous agitation à TA pendant 24 heures. La solution est filtrée et le filtrat est mis à sec puis solubilisé dans 430 ml de méthanol, 7 ml d'eau sont ajoutés suivis de 8,7 g de potasse en pastille. Le mélange est chauffé à reflux 24 heures. Après concentration, le solide obtenu est lavé à l'éther puis dissous dans le dichlorométhane. La phase organique est traitée par une solution aqueuse d'acide chlorhydrique à 10 %. La phase organique est ensuite séchée sur sulfate de magnésium puis filtrée et concentrée. On recueille 5,8 g du composé attendu sous forme d'un solide blanc, F = 194°C.

RMN¹H : δ (ppm) : 3,75 : s : 3H ; 6,9 : s : 2H ; 7,05 : dd : 2H ; 7,15-7,30 : m : 3H ; 7,45 : d : 2H ; 7,55 : dd : 1H ; 12,5 : s : 1H.

### G) 5-(4-Chlorophényl)-4-(2,4-dichlorophényl)-1-méthyl-1H-pyrrol-2-carboxamide.

4 g d'acide obtenu à l'étape précédente et 1,9 g de 1,1'-carbonyl bis(1*H-*imidazole) sont solubilisés dans 40 ml de DMF anhydre. Le mélange est agité pendant 1 heure à TA puis la solution est laissée buller dans l'ammoniac gazeux pendant 1 heure. Après ajout d'eau, le produit est extrait à l'éther éthylique. La phase organique est lavée avec une solution de NaOH(1N). Après séchage sur MgSO4, filtration et concentration on obtient 3,3 g d'un solide blanc correspondant à l'amide attendu.

LC/MS : conditions 1, MH⁺ = 379, tr = 10,42. Pureté: 95.2%.

### H) Chlorure de [5-(4-chlorophényl)-4-(2,4-dichlorophényl)-1-méthyl-1H-pyrrol-2-yl]méthanaminium.

A 8 ml d'une solution de 3.2 g du composé obtenu à l'étape précédente dans le THF est ajoutée, goutte à goutte 43ml d'une solution de [BH₃OTHF] (1N). Puis, le mélange est chauffé à 60-70°C pendant 19heures. Après refroidissement à 0°C, on ajoute 15 ml de méthanol. Le mélange est concentré au trois-quarts et la liqueur restante est ajoutée goutte à goutte à 200ml d'une solution d'éther chlorhydrique. Après précipitation et filtration , on obtient 2,55 g du composé attendu sous forme d'un solide blanc.

RMN(1H) : 2,1 ppm : s : 3H ; 3,9 ppm : s : 2H ; 6,55 ppm : s : 1H ; 7,05-7,55 ppm : m : 7H ; 8,35 ppm : s : 3H.

LC/MS : MH⁺ = 364, tr = 9,04.

### EXEMPLE 1 : Composé N° 1

### N-((5-(4-Chlorophényl)-4-(2,4-dichlorophényl)-1-méthyl-1H-pyrrol-2-yl)méthyl)-4-(trifluorométhyl)benzamide.

On solubilise 1 g du composé de la Préparation 1 dans 30 ml de DCM, en présence de 1 ml de triéthylamine. On ajoute 0,57 ml de chlorure d'acide 4-trifluorométhylbenzoïque et on laisse 24 heures sous agitation à TA. On évapore à sec puis on purifie par chromatographie pour obtenir 0,42 g du composé attendu sous forme solide.

### EXEMPLE 2 : Composé N° 2

### 3-Chloro-N-((5-(4-chlorophényl)-4-(2,4-dichlorophényl)-1-méthyl-1H-pyrrol-2-yl)méthyl)benzènesulfonamide.

On solubilise 0,5 g du composé de la Préparation 1 dans 15 ml de DCM en présence de 0,4 ml de triéthylamine ; on ajoute 0,32 mg de 3-chlorobenzènesulfonyle et on laisse sous agitation à TA pendant 20 heures. On évapore à sec puis on purifie par chromatographie pour obtenir 0,15 g du composé attendu sous forme solide.

### EXEMPLE 3 : Composé N° 5

### N-((5-(4-Chlorophényl)-4-(2,4-dichlorophényl)-1-méthyl-1H-pyrrol-2-yl)méthyl)-N'-(3-(trifluorométhyl)phényl)urée.

On solubilise 0,8 g du composé de la Préparation 1 dans 40 ml de DCM en présence de 0,40 ml de triéthylamine ; on ajoute 0,34 ml de 3-trifluorométhylbenzène isocyanate et on laisse sous agitation à TA pendant 20 heures. On purifie par chromatographie pour obtenir 0,25 g du composé attendu sous forme solide.

### EXEMPLE 4 : Composé N° 6 à 18

Les composés de formule (IA) sont préparés par chimie combinatoire selon le procédé décrit ci-après :
les acides carboxyliques de formule (III) sont dissous dans le DMF à la concentration de 0,25M en présence de 3 équivalents de DIPEA. Dans chaque puits de 2 ml, on place 120 µl de cette solution et 120 µl d'une solution de TBTU dans le DMF à la concentration de 0,25M. On ajoute dans chaque puits 300 µl d'une solution contenant la méthyamine dans le DMF à la concentration 0,1M et 3 équivalents de DIPEA. Les plaques sont agitées à TA pendant 16 heures puis évaporées. Les produits formés sont dissous dans chaque puits par 500 µl d'AcOEt, on ajoute 400 µl de Na₂CO₃ 0,1M et les plaques sont agitées. Après décantation 430 µl de phase aqueuse sont écartés puis 300 µl de NaCl à 5 % sont additionnés et les plaques sont agitées. On écarte ensuite 350 µl de phase aqueuse et les résidus sont analysés par LC/UV/MS.

Le tableau qui suit illustre les structures chimiques et les propriétés physiques des composés selon l'invention. Dans ce tableau Me, Et, Pr, tBu illustrent respectivement les groupes méthyle, éthyle, propyle, *tert*-butyle.

**TABLEAU 1**

| | | | | | | |
|---|---|---|---|---|---|---|
| 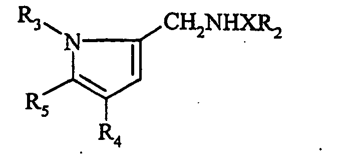 | | | | | | |

| Composés N° | X | R₂ | R₃ | R₄ | R₅ | Caractérisation / Conditions |
|---|---|---|---|---|---|---|
| 1 | -CO- | 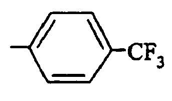 | Me | 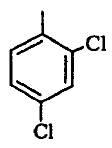 | 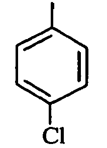 | F = 82°C MH⁺ = 537 tr = 12,27 A |
| 2 | -SO₂- | 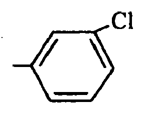 | Me | 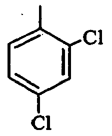 | 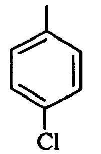 | F = 70°C MH = 537 tr = 11.86 A |
| 3 | -SO₂- | 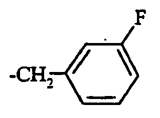 | Me | 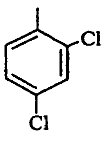 | 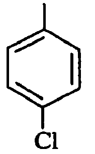 | F = 80°C MH⁺ = 537 tr = 12,03 A |
| 4 | -SO2- | 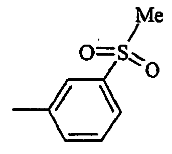 | Me | 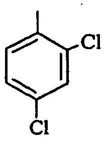 | 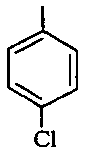 | F = 106°C MH⁺ = 583 tr= 11,19 A |
| 5 | -CONH- | 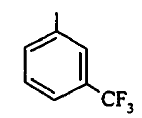 | Me | 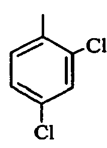 | 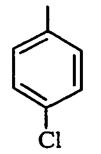 | F = 105°C MH⁺ = 552 tr = 12,23 A |
| 6 | -CO- | 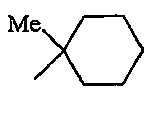 | Me | 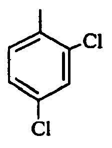 | 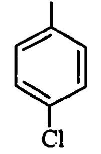 | MH⁺ = 489,6 tr=2,12 MS2 |
| 7 | -CO- | 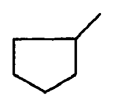 | Me | 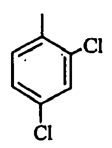 | 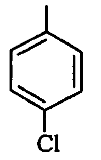 | MH⁺ = 461,5 tr = 2,03 MS2 |
| 8 | -CO- | 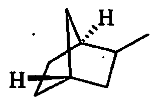 endo(racémique) | Me | 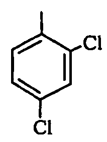 | 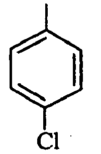 | MH⁺ = 487,6 tr = 2,10 MS2 |
| 9 | -CO- | 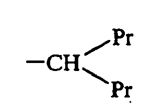 | Me | 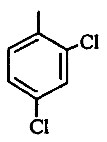 | 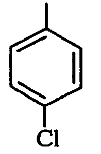 | MH⁺ = 491,6 tr = 2,13 MS2 |
| 10 | -CO- | 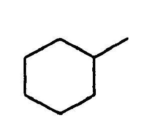 | Me | 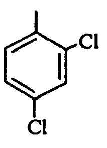 | 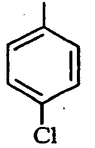 | MH⁺ = 475,6 tr = 2,06 MS2 |
| 11 | -CO- | 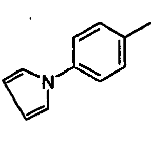 | Me | 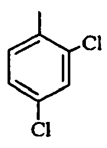 | 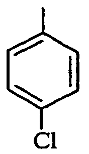 | MH⁺ = 534,5 tr=2,12 MS2 |
| 12 | -CO- | 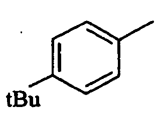 | Me | 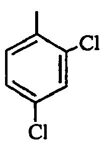 | 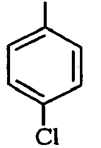 | MH⁺ = 525,6 tr=2,19 MS2 |
| 13 | -CO- | 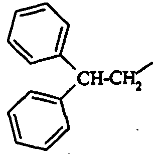 | Me | 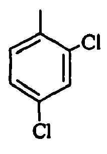 | 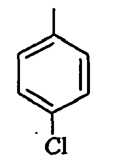 | MH⁺ = 573,5 tr=2,11 MS2 |
| 14 | -CO- | 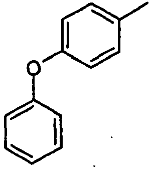 | Me | 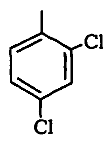 | 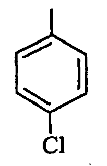 | MH⁺ = 561,5 tr=2,15 MS2 |
| 15 | -CO- | 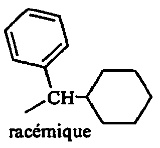 | Me | 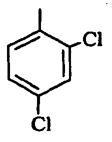 | 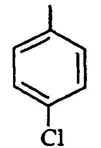 | MH⁺ = 565,6 tr = 2,25 MS2 |
| 16 | CO- | 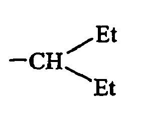 | Me | 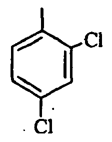 | 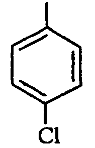 | MH⁺ = 463,6 tr = 2,03 MS2 |
| 17 | -CO- | 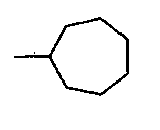 | Me | 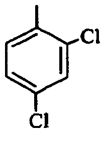 | 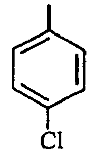 | MH⁺ = 489,6 tr = 2,10 MS2 |
| 18 | -CO- | -tBu | Me | 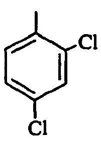 | 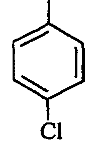 | MH⁺ = 449,6 tr = 2,01 MS2 |

Les composés de formule (I) possèdent une très bonne affinité *in vitro* (IC₅₀ ≤ 5.10⁻⁷M) pour les récepteurs aux cannabinoïdes CB₁, dans les conditions expérimentales décrites par M. Rinaldi-Carmona et al. (FEBS Letters, 1994, 350, 240-244).

La nature antagoniste des composés de formule (I) a été démontrée par les résultats obtenus dans les modèles de l'inhibition de l'adénylate-cyclase comme décrits dans M. Bouaboula et al., J. Biol. Chem., 1995, 270, 13973-13980, M. Rinaldi-Carmona et al., J. Pharmacol. Exp. Ther., 1996, 278, 871-878 et M. Bouaboula et al., J. Biol. Chem., 1997, 272, 22330-22339.

L'interaction d'un composé selon l'invention avec les récepteurs CB1 présents dans le cerveau est déterminée chez la souris avec le test de binding ex vivo du [3H]-CP55940 après une injection intraveineuse comme décrit dans Rinaldi-Carmona M et al FEBS Letters (1994), 350, 240-244 et Rinaldi-Carmona M et al., Life Sciences (1995), 56, 1941-1947.

L'interaction d'un composé selon l'invention avec les récepteurs CB1 présents à la périphérie est déterminée chez la souris avec le test de réversion de l'effet inhibiteur du CP55940 sur transit gastrointestinal après une administration orale comme décrit dans Rinaldi-Carmona M et al., JPET (2004), 310, 905-914.

La toxicité des composés de formule (I) est compatible avec leur utilisation en tant que médicament.

Ainsi, selon un autre de ses aspects, l'invention a pour objet des médicaments pour la médecine humaine ou vétérinaire qui comprennent un composé de formule (I), ou un sel d'addition de ce dernier à un acide pharmaceutiquement acceptable, ou encore un solvat ou un hydrate du composé de formule (I).

Ainsi les composés selon l'invention peuvent être utilisés dans le traitement ou la prévention de maladies impliquant les récepteurs aux cannabinoïdes CB₁, chez l'homme ou chez l'animal notamment chez les mammifères incluant de façon non limitative les chiens, les chats, les chevaux, les bovins, les moutons.

Par exemple et de manière non limitative, les composés de formule (I) sont utiles comme médicaments psychotropes, notamment pour le traitement des désordres psychiatriques incluant l'anxiété, la dépression, les troubles de l'humeur, l'insomnie, les troubles délirants, les troubles obsessionnels, les psychoses en général, la schizophrénie, les troubles de l'attention et de l'hyperactivité (TDAH) chez les enfants hyperkinétiques ainsi que pour le traitement des troubles liés à l'utilisation de substances psychotropes, notamment dans le cas d'un abus d'une substance et/ou de dépendance à une substance, y compris la dépendance alcoolique et la dépendance nicotinique.

Les composés de formule (I) selon l'invention peuvent être utilisés comme médicaments pour le traitement de la migraine, du stress, des maladies d'origine psychosomatique, des crises d'attaques de panique, de l'épilepsie, des troubles du mouvement, en particulier des dyskinésies ou de la maladie de Parkinson, des tremblements et de la dystonie.

Les composés de formule (I) selon l'invention peuvent également être utilisés comme médicaments dans le traitement des troubles mnésiques, des troubles cognitifs, en particulier dans le traitement des démences séniles, de la maladie d'Alzheimer, ainsi que dans le traitement des troubles de l'attention ou de la vigilance. De plus, les composés de formule (I) peuvent être utiles comme neuroprotecteurs, dans le traitement de l'ischémie, des traumatismes crâniens et le traitement des maladies neurodégénératives aiguës ou chroniques : incluant la chorée, la chorée de Huntington, le syndrome de Tourrette.

Les composés de formule (I) selon l'invention peuvent être utilisés comme médicaments dans le traitement de la douleur : les douleurs neuropathiques, les douleurs aiguës périphériques, les douleurs chroniques d'origine inflammatoire, les douleurs induites par un traitement anticancéreux.

Les composés de formule (I) selon l'invention peuvent être utilisés comme médicaments dans la médecine humaine ou vétérinaire dans le traitement et la prévention des troubles de l'appétit, de l'appétence (pour les sucres, carbohydrates, drogues, alcools ou toute substance appétissante) et/ou des conduites alimentaires, notamment pour le traitement de l'obésité ou de la boulimie ainsi que pour le traitement du diabète de type II ou diabète non insulinodépendant et pour le traitement des dyslipidémies, du syndrome métabolique. Ainsi les composés de formule (I) selon l'invention sont utiles dans le traitement et la prévention de l'obésité et des risques associés à l'obésité, notamment les risques cardio-vasculaires.

De plus, les composés de formule (I) selon l'invention peuvent être utilisés en tant que médicaments dans le traitement et la prévention des troubles gastro-intestinaux, des troubles diarrhéiques, des ulcères, des vomissements, des troubles vésicaux et urinaires, des maladies du foie telles que la cirrhose chronique, la fibrose, la stéatose hépatique, la stéatohépatite ; ainsi que des troubles d'origine endocrinienne, des troubles cardio-vasculaires, de l'hypotension, de l'athérosclérose, du choc hémorragique, du choc septique, de l'asthme, de la bronchite chronique, des maladies pulmonaires chroniques obstructives, du syndrome de Raynaud, du glaucome, des troubles de la fertilité, de l'accouchement prématuré, des phénomènes inflammatoires, des maladies du système immunitaire, en particulier autoimmunes et neuroinflammatoires tel que l'arthrite rhumatoïde, l'arthrite réactionnelle, les maladies entraînant une démyélinisation, la sclérose en plaque, des maladies infectieuses et virales telles que les encéphalites, des accidents vasculaires cérébraux et en tant que médicaments pour la chimiothérapie anticancéreuse, pour le traitement du syndrome de Guillain-Barré et pour le traitement des maladies des os et de l'ostéoporose.

Selon la présente invention, les composés de formule (I) sont tout particulièrement utiles pour la préparation de médicaments utiles pour la prévention et le traitement des troubles psychotiques, en particulier la schizophrénie, les troubles de l'attention et de l'hyperactivité (TDAH) chez les enfants hyperkinétiques ; des déficits mnésiques et cognitifs ; de la dépendance alcoolique, de la dépendance nicotinique, du sevrage alcoolique et du sevrage tabagique, des maladies neurodégénératives aiguës ou chroniques.

Plus particulièrement, les composés de formule (I) selon la présente invention sont utiles dans le traitement et la prévention des troubles de l'appétit, des troubles métaboliques, des troubles gastro-intestinaux, des phénomènes inflammatoires, des maladies du système immunitaire, des troubles psychotiques, de la dépendance alcoolique, de la dépendance nicotinique.

Selon un de ses aspects, la présente invention est relative à l'utilisation d'un composé de formule (I), de ses sels pharmaceutiquement acceptables et de leurs solvats ou hydrates pour le traitement des troubles et maladies indiqués ci-dessus.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, un solvat ou hydrate dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Les compositions pharmaceutiques selon la présente invention peuvent contenir à côté d'un composé de formule (I) un (ou plusieurs) autre principe actif utile dans le traitement des troubles et maladies indiquées ci-dessus.

Ainsi, la présente invention a également pour objet des compositions pharmaceutiques contenant un composé de formule (I) selon la présente invention associé à un (ou plusieurs) principe actif choisi parmi l'une des classes thérapeutiques suivantes :
- un autre antagoniste des récepteurs CB₁ aux cannabinoïdes ;
- un modulateur des récepteurs CB₂ aux cannabinoïdes ;
- un antagoniste des récepteurs AT₁ de l'angiotensine II ;
- un inhibiteur de l'enzyme de conversion ;
- un antagoniste calcique ;
- un diurétique ;
- un béta-bloquant ;
- un antihyperlipémiant ou un antihypercholestérolémiant ;
- un antidiabétique ;
- un autre agent anti-obésité ;
- un agoniste nicotinique, un agoniste nicotinique partiel ;
- un antidépresseur, un antispychotique, un anxiolytique ;
- un anticancéreux ou un agent antiprolifératif ;
- un antagoniste des opioïdes ;
ainsi que :
- un agent améliorant la mémoire ;
- un agent utile dans le traitement de l'alcoolisme ou des symptômes de sevrage ;
- un agent utile pour traiter l'ostéoporose ;
- un anti-inflammatoire non stéroïdien ou stéroïdien ;
- un anti-infectieux ;
- un analgésique ;
- un antiasthmatique.

Par antagoniste des récepteurs AT₁ de l'angiotensine II, on entend un composé tel que candésartan cilexitil, éprosartan, irbésartan, losartan potassium, olmésartan médoxomil, telmisartan, valsartan, chacun de ces composés pouvant être lui-même associé à un diurétique tel que l'hydrochlorothiazide.

Par inhibiteur de l'enzyme de conversion, on entend un composé tel que alacépril, bénazépril, captopril, cilazapril, énalapril, énalaprilat, fosinopril, imidapril, lisinopril, moexipril, périndopril, quinapril, ramipril, spirapril, temocapril, trandolapril, zofénopril, chacun de ces composés pouvant lui-même être associé à un diurétique tel que l'hydrochlorothiazide ou l'indapamide ou à un antagoniste calcique tel que l'amlodipine, le diltiazem, le félodipine ou le vérapamil.

Par antagoniste calcique, on entend un composé tel que amlodipine, aranidipine, bénidipine, bépridil, cilnidipine, diltiazem, éfonidipine hydrochloride éthanol, fasudil, félodipine, isradipine, lacidipine, lercanidipine hydrochloride, manidipine, mibéfradil hydrochloride, nicardipine, nifédipine, nilvadipine, nimodipine, nisoldipine, nitrendipine, térodiline, vérapamil.

Par béta-bloquant, on entend un composé tel que acébutolol, alprénolol, amosulalol, arotinolol, aténolol, béfunolol, bétaxolol, bévantolol, bisoprolol, bopindolol, bucumolol, bufétolol, bunitrolol, butofilolol, carazolol, cartéolol, carvédilol, cloranolol, épanolol, esmolol, indénolol, labétalol, landiolol, lévobunolol, lévomoprolol, mépindolol, métipranolol, métoprolol, nadolol, nébivolol, nifénalol, nipradilol, oxprénolol, penbutolol, pindolol, propranolol, salmétérol, sotalol, talinolol, tertatolol, tilisolol, timolol, xamotérol, xibénolol.

Par antihyperlipémiant ou antihypercholestérolémiant, on entend un composé choisi parmi les fibrates tels que alufibrate, béclobrate, bézafibrate, ciprofibrate, clinofibrate, clofibrate, étofibrate, fénofibrate ; les statines (inhibiteurs de HMG-CoA reductase), telles que atorvastatine, fluvastatine sodium, lovastatine, pravastatine; rosuvastatine, simvastatine, ou un composé tel que acipimox, aluminum nicotinate, azacostérol, cholestyramine, dextrothyroxine, méglutol, nicéritrol, nicoclonate, acide nicotinique, béta-sitosterol, tiadénol.

Par antidiabétique, on entend un composé appartenant à l'une des classes suivantes : les sulfonylurées, les biguanidines, les inhibiteurs d'alpha glucosidase, les thiazolidinedione, les métiglinides, tel que acarbose, acétohexamide, carbutamide, chlorpropamide, glibenclamide, glibornuride, gliclazide, glimépiride, glipizide, gliquidone, glisoxepide, glybuzole, glymidine, métahexamide, métformin, miglitol, natéglinide, pioglitazone, répaglinide, rosiglitazone, tolazamide, tolbutamide, troglitazone, voglibose, ainsi que l'insuline et les analogues de l'insuline.

Par autre agent anti-obésité, on entend un composé tel que amfépramone, benfluorex, benzphétamine, indanorex, mazindole, méfénorex, méthamphétamine, D-norpseudoéphédrine, sibutramine, un topiramate, un inhibiteur de lipase (orlistat cetilistat), un agoniste PPAR (de l'anglais Peroxisome Proliferator Activated Receptor Agonist), un agoniste de la dopamine, un agoniste des récepteurs de leptine, un inhibiteur du reuptake de la sérotonine, un béta-3 agoniste, un CCK-A agoniste, un inhibiteur de NPY,un agoniste des récepteurs MC4, un antagoniste des récepteurs MCH (de l'anglais Melanin Concentrating Hormone), un antagoniste de l'orexine, un inhibiteur de phosphodiesterase, un inhibiteur de 11βHSD (11-β-hydroxy steroid deshydrogenase), un inhibiteur de DPP-IV (dipeptidyl peptidase IV), un antagoniste (ou agoniste inverse de l'histamine H3, un dérivé CNTF (de l'anglais Ciliary Neurotrophic Factor), un agoniste des récepteurs GHS (de l'anglais Growth Hormone Secretagogue, un modulateur de la ghréline, un inhibiteur de diacyglycerol acyltransferase (DGAT), un inhibiteur de phosphodiesterase (PDE), un agoniste d'hormone thyroïdienne, un antagoniste des récepteurs glucocorticoïdes, un inhibiteur de stearoyl-CoA- desaturase (SCD), un inhibiteur des transporteurs de phosphate, de glucose, d'acide gras, de dicarboxylate, un antagoniste 5HT₂, un antagoniste 5HT₆, un agoniste de la bombesine.

Par antagoniste des opioïdes on entend un composé tel que naltrexone, naloxone ou nalméfène.

Par agent utile dans le traitement de l'alcoolisme ainsi que des symptômes de sevrage on entend l'acamprosate, les benzodiazepines, les béta-bloquants, la clonidine, la carbamazépine.

Par agent utile, pour traiter l'ostéoporose, on entend par exemple, les biphosphonates tels que étidronate, clodronate, tiludronate, risédronate.

Selon la présente invention, on peut également associer d'autres composés ayant des propriétés antihyperlipemiantes, antihypercholesterolemiantes, antidiabétiques ou anti-obésité. Plus particulièrement on peut associer des composés appartenant à l'une des classes suivantes :
inhibiteurs de PTP 1B (de l'anglais Protein Tyrosine Phosphase -1B), les agonistes des récepteurs VPAC 2, les modulateurs de GLK, les modulateurs retinoides, les inhibiteurs de glycogen phosporylase (HGLPa), les antagonistes du glucagon, les inhibiteurs de glucose-6 phosphate, les activateurs de pyruvate dehydrogenase kinase (PKD), les modulateurs de RXR, FXR, LXR, les inhibiteurs de SGLT (de l'anglais Sodium Dependant Glucose Transporter), les inhibiteurs de CETP (de l'anglais Cholesterylester Transfer Protein), les inhibiteurs de squalene synthetase, les inhibiteurs de squalene epoxidase, les inhibiteurs de synthèse des triglycérides, les inducteurs de récepteurs LDL (de l'anglais Low Density Lipoprotein), les inhibiteurs de IBAT, les inhibiteurs de FBPase (fructose-1,6-biphosphatase), les modulateurs de CART (de l'anglais Cocaïne-Amphétamine-Regulated Transcript),
les modulateurs MC 4 (mélanocortin 4), les antagonistes des récepteurs de l'oréxine.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | : 50,0 mg |
| Mannitol | : 223,75 mg |
| Croscarmellose sodique | : 6,0 mg |
| Amidon de maïs | : 15,0 mg |
| Hydroxypropyl-méthylcellulose | : 2,25 mg |
| Stéarate de magnésium | : 3,0 mg |

Par voie orale, la dose de principe actif administrée par jour peut atteindre 0,01 à 100 mg/kg, en une ou plusieurs prises, préférentiellement 0,02 à 50 mg/kg.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvats.

## Revendications

1. Composé répondant à la formule (I) : 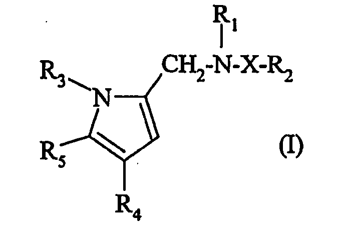 dans laquelle :
- X représente un groupe 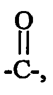 -SO₂-, 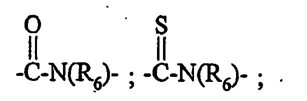
- R₁ représente un atome d'hydrogène ou un groupe (C₁-C₄)alkyle ;
- R₂ représente :
. un (C₁-C₁₂)alkyle non substitué ou substitué une ou plusieurs fois par des substituants choisis indépendamment parmi un atome d'halogène, un hydroxyle, un (C₁-C₄) alcoxy, un (C₁-C₄)alkylthio, un phénoxy, un radical trifluorométhoxy, un radical difluorométhoxy, un radical difluorométhylthio, un radical trifluorométhylthio ;
. un radical carbocyclique non aromatique en (C₃-C₁₂) non substitué ou substitué une ou plusieurs fois par des substituants choisis indépendamment parmi un (C₁-C₄)alkyle, un (C₁-C₄)alcoxy, un (C₁-C₄)alkylthio, un atome de fluor, un hydroxyle, un radical trifluorométhyle, difluorométhyle, trifluorométhoxy, difluorométhoxy, trifluorométhylthio, difluorométhylthio ;
. un méthyle substitué par un radical carbocyclique non aromatique en (C₃-C₁₂) non substitué ou substitué une ou plusieurs fois par des substituants choisis indépendamment parmi un (C₁-C₄)alkyle, un (C₁-C₄)alcoxy, un (C₁-C₄)alkylthio, un atome de fluor, un hydroxyle, un radical trifluorométhyle, difluorométhyle, trifluorométhoxy, difluorométhoxy, trifluorométhylthio, difluorométhylthio ;
. un radical phényle, benzyle, benzhydryle, benzydrylméthyle, dans lequel chaque groupe phényle est non substitué ou substitué une ou plusieurs fois par des substituants choisis indépendamment parmi un atome d'halogène, un hydroxyle, un (C₁-C₄)alkyle, un (C₁-C₄)alcoxy, un méthylènedioxy, un cyano, un nitro, un trifluorométhyle, un difluorométhyle, un difluorométhoxy, un trifluorométhoxy, un trifluorométhylthio, un difluorométhylthio, un groupe S(O)ₙAlk, un groupe OS(O)ₙAlk, un groupe (C₁-C₄)alkylcarbonyle ;
. un radical phényle substitué par un radical hétérocyclique choisi parmi pyrrolyle, imidazolyle, pyridyle ou pyrrazolyle, ledit radical hétérocyclique étant non substitué ou substitué une ou plusieurs fois par un ou plusieurs substituants choisis indépendamment parmi un atome d'halogène ou un groupe (C₁-C₄)alkyle ;
. un radical phényle substitué par un phényle ou un phénoxy dans lequel chaque groupe phényle est non substitué ou substitué une ou plusieurs fois par des substituants choisis indépendamment parmi un atome d'halogène, un hydroxyle, un (C₁-C₄)alkyle, un (C₁-C₄)alcoxy, un méthylènedioxy, un cyano, un nitro, un trifluorométhyle, un difluorométhyle, un difluorométhoxy, un trifluorométhoxy, un trifluorométhylthio, un difluorométhylthio, un groupe S(O)ₙAlk, un groupe OS(O)ₙAlk, un groupe (C₁-C₄)alkylcarbonyle ;
. un 1,2,3,4-tétrahydronaphtalèn-2-yle non substitué ou substitué une ou plusieurs fois par un (C₁-C₄)alkyle ou un trifluorométhyle ;
. un radical pyrrolyle, imidazolyle, pyridyle, pyrazolyle, furyle, thiényle, oxazolyle, thiazolyle, lesdits radicaux étant non substitués ou substitués par un ou plusieurs substituants choisis indépendamment parmi un atome d'halogène, un groupe (C₁-C₄)alkyle ou trifluorométhyle ;
. un indol-2-yle ou un N-méthylindol-2-yle ;
- R₃ représente un (C₁-C₅)alkyle ou un (C₃-C₇) cycloalkyle ;
- R₄ représente un phényle non substitué ou substitué une ou plusieurs fois par des substituants choisis indépendamment parmi un atome d'halogène, un (C₁-C₄)alkyle, un (C₁-C₄)alcoxy, un cyano, un radical difluorométhyle, trifluorométhyle, difluorométhoxy, trifluorométhoxy, un groupe S(O)ₙAlk ou OS(O)ₙAlk ;
- R₅ représente un phényle non substitué ou substitué une ou plusieurs fois par des substituants choisis indépendamment parmi un atome d'halogène, un (C₁-C₄)alkyle, un (C₁-C₄)alcoxy, un cyano, un radical difluorométhyle, trifluorométhyle, difluorométhoxy, trifluorométhoxy, un groupe S(O)ₙAlk ou OS(O)ₙAlk ;
- R₆ représente un atome d'hydrogène ou un (C₁-C₄)alkyle ;
- n représente 0, 1 ou 2 ;
- Alk représente un (C₁-C₄)alkyle ;
à l'état de base ou de sel d'addition à des acides ainsi qu'à l'état d'hydrates ou de solvats.

2. Composé répondant à la formule (I), selon la revendication 1 dans laquelle :
- X représente un groupe 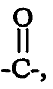 -SO₂-, 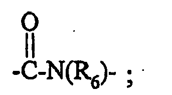
- R₁ représente un atome d'hydrogène ou un groupe (C₁-C₄)alkyle ;
- R₂ représente :
. un (C₁-C₇)alkyle ;
. un radical carbocyclique non aromatique en C₃-C₁₂ non substitué ou substitué une ou plusieurs fois par un groupe (C₁-C₄)alkyle ;
. un méthyle substitué par un radical carbocyclique non aromatique en C₃-C₁₂ et non substitué ou substitué une ou plusieurs fois sur le carbocycle par un (C₁-C₄)alkyle ;
. un phényle non substitué ou substitué une ou plusieurs fois par des substituants choisis indépendamment parmi un atome d'halogène, un (C₁-C₄)alkyle, un hydroxyle, un (C₁-C₄)alcoxy, un cyano, un groupe trifluorométhyle, un groupe trifluorométhoxy, un groupe S(O)ₙAlk, un groupe (C₁-C₄)alkylcarbonyle ; ou parmi un radical phényle, phénoxy, pyrrolyle, imidazolyle, pyridyle ou pyrazolyle, lesdits radicaux étant non substitués ou substitués une ou plusieurs fois par un (C₁-C₄)alkyle ;
. un benzyle non substitué ou substitué une ou plusieurs fois sur le phényle par des substituants choisis indépendamment parmi un atome d'halogène, un cyano, un (C₁-C₄)alkyle, un (C₁-C₄)alcoxy, un radical trifluorométhyle ou un groupe S(O)ₙAlk ; ou substitué en alpha par un ou deux groupes semblables ou différents choisis parmi un (C₁-C₄)alkyle et un (C₃-C₇)cycloalkyle ;
. un benzhydryle, un groupe benzhydrylméthyle ;
. un 1,2,3,4-tétrahydronaphtalèn-2-yle non substitué ou substitué une ou plusieurs fois par un (C₁-C₄)alkyle ;
. un radical pyrrolyle, imidazolyle, pyridyle, pyrazolyle, furyle, thiényle, lesdits radicaux étant non substitués ou substitués par un ou plusieurs substituants choisis indépendamment parmi un atome d'halogène ou un groupe (C₁-C₄)alkyle ;
. un indol 2-yle ou un N-méthylindol-2-yle ;
- R₃ représente un (C₁-C₅)alkyle ou un (C₃-C₇) cycloalkyle ;
- R₄ représente un phényle non substitué ou substitué une ou plusieurs fois par des substituants choisis indépendamment parmi un atome d'halogène, un (C₁-C₄)alkyle, un (C₁-C₄)alcoxy, un radical trifluorométhyle ou un groupe S(O)ₙAlk ;
- R₅ représente un phényle non substitué ou substitué une ou plusieurs fois par des substituants choisis indépendamment parmi un atome d'halogène, un (C₁-C₄)alkyle, un (C₁-C₄)alcoxy, un radical trifluorométhyle ou un groupe S(O)ₙAk ;
- R₆ représente un atome d'hydrogène ou un (C₁-C₄)alkyle ;
- n représente 0, 1 ou 2 ;
- Alk représente un (C₁-C₄)alkyle ;
à l'état de base ou de sel d'addition à des acides ainsi qu'à l'état d'hydrates ou de solvats.

3. Composé, selon la revendication 1 de formule (IA) dans laquelle -X- représente un groupe -CO- et les substituants R₁ à R₅ sont tels que définis pour les composés de formule (I) à la revendication 1.

4. Composé, selon la revendication 1 de formule (IB) dans laquelle -X- représente un groupe -SO₂- et les substituants R₁ à R₅ sont tels que définis pour les composés de formule (I) à la revendication 1.

5. Composé, selon la revendication 1 de formule (IC) dans laquelle -X- représente un groupe -CON(R₆)- et les substituants R₁ à R₆ sont tels que définis pour les composés de formule (I) à la revendication 1.

6. Procédé pour la préparation d'un composé de formule (I) selon la revendication 1 **caractérisé en ce que** :
on traite un composé de formule : 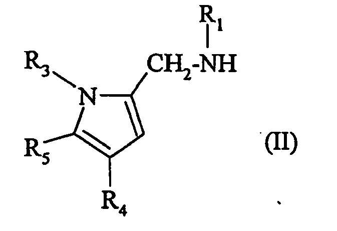 dans laquelle R₁, R₃, R₄ et R₅ sont tels que définis pour un composé de formule (I) :
- soit par un acide ou un dérivé fonctionnel de cet acide de formule :
HOOC-R₂ (III)
dans laquelle R₂ est tel que défini pour un composé de formule (IA), lorsqu'on doit préparer un composé de formule (IA) dans laquelle -X- représente un groupe -CO- ;
- soit par un halogénure de sulfonyle de formule :
Hal-SO₂-R₂ (IV)
dans laquelle R₂ est tel que défini pour un composé de formule (I) et Hal représente un atome d'halogène, préférentiellement le chlore, lorsqu'on doit préparer un composé de formule (IB) dans laquelle -X- représente un groupe-SO₂- ;
- soit par un halogénoformiate de formule :
HalCOOAr (V)
dans laquelle Hal représente un atome d'halogène et Ar représente un phényle ou un 4-nitrophényle pour obtenir un composé intermédiaire de formule : 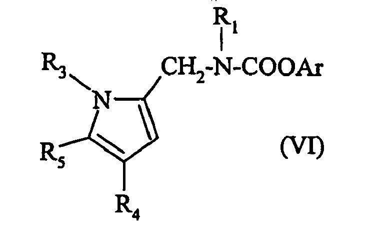 dans laquelle R₁, R₃, R₄ et R₅ sont tels que définis pour un composé de formule (I), que l'on fait réagir ensuite avec une amine de formule :
HN(R₆)R₂ (VII)
dans laquelle R₂ et R₆ sont tels que définis pour un composé de formule (I), lorsqu'on doit préparer un composé de formule (IC) dans laquelle -X- représente un groupe -CON(R₆)- ;
- soit par un isothiocyanate de formule R₂-N=C=S (IX) dans laquelle R₂ est tel que défini pour un composé de formule (I) lorsque l'on doit préparer un composé de formule I(D) dans laquelle -X- représente un groupe -CSN(R₆)-.

7. Composé de formule : 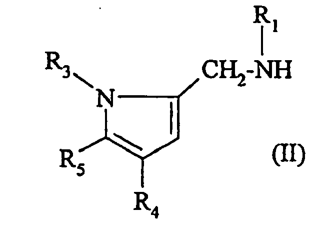 dans laquelle R₁, R₃, R₄ et R₅ sont tels que définis pour (I) à la revendication 1.

8. Médicament **caractérisé en ce qu'**il comprend un composé de formule (I) selon l'un quelconque des revendications 1 à 5, ou un hydrate ou un solvat d'un composé de formule (I).

9. Composition pharmaceutique, **caractérisée en ce qu'**elle comprend un composé de formule (I) selon l'une quelconque des revendications 1 à 5, ou un hydrate ou un solvat de ce composé, ainsi qu'au moins un excipient pharmaceutiquement acceptable.

10. Utilisation d'un composé de formule (I) tel que défini dans les revendications 1 à 5 pour la préparation d'un médicament pour le traitement ou la prévention des maladies dans lesquelles les récepteurs CB₁ sont impliqués.

11. Utilisation selon la revendication 10 **caractérisée en ce que** les maladies sont les désordres psychiatriques, la dépendance et le sevrage à une substance, les troubles cognitifs, les troubles de l'attention et de la vigilance, et les maladies neurodégénératives aiguës et chroniques.

12. Utilisation selon la revendication 10 **caractérisée en ce que** les maladies sont les troubles du métabolisme, les troubles de l'appétance, les troubles de l'appétit, l'obésité, le diabète de type II, le syndrome métabolique, la dyslipidémie.

13. Utilisation selon la revendication 10 **caractérisée en ce que** les maladies sont la douleur, la douleur neuropathique, la douleur induite par le traitement anticancéreux.

14. Utilisation selon la revendication 10 **caractérisée en ce que** les maladies sont les troubles gastro-intestinaux, les vomissements, les troubles diarrhéiques, l'ulcère, les maladies du foie.

15. Utilisation selon la revendication 10 **caractérisée en ce que** les maladies sont les maladies du système immunitaire, arthrite rhumatoïde, la démyélinisation, la sclérose en plaque, les maladies inflammatoires.

16. Utilisation selon la revendication 10 **caractérisée en ce que** les maladies sont les maladies d'Alzheimer, de Parkinson, la schizophrénie, les troubles cognitifs, le diabète, l'obésité, le syndrome métabolique et le sevrage tabagique.

## Claims

1. Compound corresponding to formula (I): 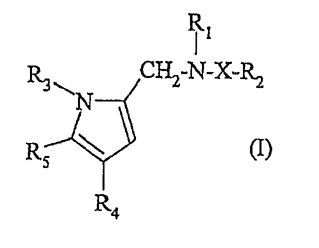 in which:
- X represents a group 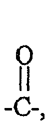 -SO₂-, 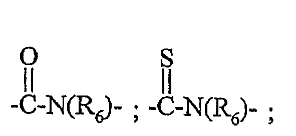
- R₁ represents a hydrogen atom or a (C₁-C₄) alkyl group;
- R₂ represents:
• a (C₁-C₁₂₎ alkyl which is unsubstituted or substituted one or more times with substituents chosen independently from a halogen atom, a hydroxyl, a (C₁-C₄) alkoxy, a (C₁-C₄) alkylthio, a phenoxy, a trifluoromethoxy radical, a difluoromethoxy radical, a difluoromethylthio radical and a trifluoromethylthio radical;
• a nonaromatic carbocyclic (C₃-C₁₂) radical which is unsubstituted or substituted one or more times with substituents chosen independently from a (C₁-C₄) alkyl, a (C₁-C₄)alkoxy, a (C₁-C₄) alkylthio, a fluorine atom, a hydroxyl, a trifluoromethyl radical, a difluoromethyl radical, a trifluoromethoxy radical, a difluoromethoxy radical, a trifluoromethylthio radical and a difluoromethylthio radical;
• a methyl substituted with a nonaromatic carbocyclic (C₃-C₁₂) radical which is unsubstituted or substituted one or more times with substituents chosen independently from a (C₁-C₄) alkyl, a (C₁-C₄) alkoxy, a (C₁-C₄) alkylthio, a fluorine atom, a hydroxyl, a trifluoromethyl radical, a difluoromethyl radical, a trifluoromethoxy radical, a difluoromethoxy radical, a trifluoromethylthio radical and a difluoromethylthio radical;
• a phenyl, benzyl, benzhydryl or benzhydrylmethyl radical, in which each phenyl group is unsubstituted or substituted one or more times with substituents chosen independently from a halogen atom, a hydroxyl, a (C₁-C₄) alkyl, a (C₁-C₄) alkoxy, a methylenedioxy, a cyano, a nitro, a trifluoromethyl, a difluoromethyl, a difluoromethoxy, a trifluoromethoxy, a trifluoromethylthio, a difluoromethylthio, an S (O) ₙAlk group, an OS (O) ₙAlk group and a (C₁-C₄) alkylcarbonyl group;
• a phenyl radical substituted with a heterocyclic radical chosen from pyrrolyl, imidazolyl, pyridyl or pyrrazolyl, said heterocyclic radical being unsubstituted or substituted one or more times with one or more substituents chosen independently from a halogen atom or a (C₁-C₄) alkyl group;
• a phenyl radical substituted with a phenyl or a phenoxy, in which each phenyl group is unsubstituted or substituted one or more times with substituents chosen independently from a halogen atom, a hydroxyl, a (C₁-C₄)alkyl, a (C₁-C₄) alkoxy, a methylenedioxy, a cyano, a nitro, a trifluoromethyl, a difluoromethyl, a difluoromethoxy, a trifluoromethoxy, a trifluoromethylthio, a difluoromethylthio, an S (O) ₙAlk group, an OS (O) ₙAlk group and a (C₁-C₄)alkylcarbonyl group;
• a 1,2,3,4-tetrahydronaphthalen-2-yl which is unsubstituted or substituted one or more times with a (C₁-C₄) alkyl or a trifluoromethyl;
• a pyrrolyl, imidazolyl, pyridyl, pyrazolyl, furyl, thienyl, oxazolyl or thiazolyl radical, said radicals being unsubstituted or substituted with one or more substituents chosen independently from a halogen atom, a (C₁-C₄)alkyl group or a trifluoromethyl group;
• an indol-2-yl or an N-methylindol-2-yl;
- R₃ represents a (C₁-C₅) alkyl or a (C₃-C₇)cycloalkyl;
- R₄ represents a phenyl which is unsubstituted or substituted one or more times with substituents chosen independently from a halogen atom, a (C₁-C₄) alkyl, a (C₁-C₄) alkoxy, a cyano, a difluoromethyl radical, a trifluoromethyl radical, a difluoromethoxy radical, a trifluoromethoxy radical, an S(O)ₙAlk group or an OS(O)ₙAlk group;
- R₅ represents a phenyl which is unsubstituted or substituted one or more times with substituents chosen independently from a halogen atom, a (C₁-C₄) alkyl, a (C₁-C₄) alkoxy, a cyano, a difluoromethyl radical, a trifluoromethyl radical, a difluoromethoxy radical, a trifluoromethoxy radical, an S(O)ₙAlk group or an OS(O)ₙAlk group;
- R₆ represents a hydrogen atom or a (C₁-C₄) alkyl;
- n represents 0, 1 or 2;
- Alk represents a (C₁-C₄) alkyl;
in the form of a base or of an addition salt with acids, and also in the form of hydrates or solvates.

2. Compound corresponding to formula (I), according to Claim 1, in which:
- X represents a group 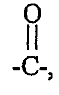 -SO₂-, 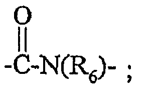
- R₁ represents a hydrogen atom or a (C₁-C₄) alkyl group;
- R₂ represents:
• a (C₁-C₇) alkyl;
• a nonaromatic carbocyclic C₃-C₁₂ radical which is unsubstituted or substituted one or more times with a (C₁-C₄) alkyl group;
• a methyl substituted with a nonaromatic carbocyclic C₃-C₁₂ radical which is unsubstituted or substituted one or more times on the carbocycle with a (C₁-C₄) alkyl;
• a phenyl which is unsubstituted or substituted one or more times with substituents chosen independently from a halogen atom, a (C₁-C₄)alkyl , a hydroxyl, a (C₁-C₄) alkoxy, a cyano, a trifluoromethyl group, a trifluoromethoxy group, an S(O)ₙAlk group and a (C₁-C₄)alkylcarbonyl group; or from a phenyl, phenoxy, pyrrolyl, imidazolyl, pyridyl or pyrazolyl radical, said radicals being unsubstituted or substituted one or more times with a (C₁-C₄) alkyl;
• a benzyl which is unsubstituted or substituted one or more times on the phenyl with substituents chosen independently from a halogen atom, a cyano, a (C₁-C₄) alkyl, a (C₁-C₄)alkoxy, a trifluoromethyl radical or an S(O)ₙAlk group; or substituted in the alpha-position with one or two similar or different groups chosen from a (C₁-C₄) alkyl and a (C₃-C₇)cycloalkyl;
• a benzhydryl or a benzhydrylmethyl group;
• a 1,2,3,4-tetrahydronaphthalen-2-yl which is unsubstituted or substituted one or more times with a (C₁-C₄) alkyl;
• a pyrrolyl, imidazolyl, pyridyl, pyrazolyl, furyl or thienyl radical, said radicals being unsubstituted or substituted with one or more substituents chosen independently from a halogen atom or a (C₁-C₄) alkyl group;
• an indol-2-yl or an N-methylindol-2-yl;
- R₃ represents a (C₁-C₅)alkyl or a (C₃-C₇ cycloalkyl;
- R₄ represents a phenyl which is unsubstituted or substituted one or more times with substituents chosen independently from a halogen atom, a (C₁-C₄)alkyl, a (C₁-C₄) alkoxy, a trifluoromethyl radical or an S(O)ₙAlk group;
- R₅ represents a phenyl which is unsubstituted or substituted one or more times with substituents chosen independently from a halogen atom, a (C₁-C₄) alkyl, a (C₁-C₄) alkoxy, a trifluoromethyl radical or an S(O)ₙAlk group;
- R₆ represents a hydrogen atom or a (C₁-C₄) alkyl;
- n represents 0, 1 or 2;
- Alk represents a (C₁-C₄) alkyl;
in the form of a base or of an addition salt with acids and also in the form of hydrates or solvates.

3. Compound, according to Claim 1, of formula (IA) in which -X- represents a -CO- group and the substituents R₁ to R₅ are as defined for the compounds of formula (I) in claim 1.

4. Compound, according to Claim 1, of formula (IB) in which -X- represents an -SO₂- group and the substituents R₁ to R₅ are as defined for the compounds of formula (I) in claim 1.

5. Compound, according to Claim 1, of formula (IC) in which -X- represents a -CON(R₆)- group and the substituents R₁ to R₆ are as defined for the compounds of formula (I) in claim 1.

6. Method for preparing a compound of formula (I) according to Claim 1, **characterized in that**:
a compound of formula: 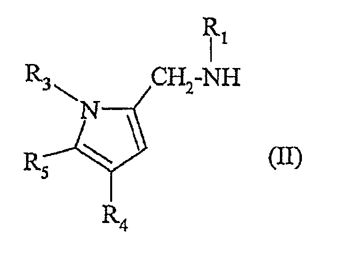 in which R₁, R₃, R₄ and R₅ are as defined for a compound of formula (I), is treated:
- either with an acid or a functional derivative of this acid of formula:
HOOC-R₂ (III)
in which R₂ is as defined for a compound of formula (IA), when a compound of formula (IA) in which -X-represents a -CO- group must be prepared;
- or with a sulfonyl halide of formula:
Hal-SO₂-R₂ (IV)
in which R₂ is as defined for a compound of formula (I) and Hal represents a halogen atom, preferably chlorine, when a compound of formula (IB) in which -X- represents an -SO₂- group must be prepared;
or with a haloformate of formula:
HalCOOAr (V)
in which Hal represents a halogen atom and Ar represents a phenyl or a 4-nitrophenyl, so as to obtain an intermediate compound of formula: 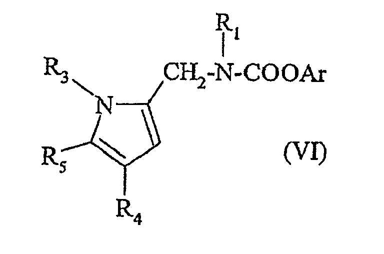 in which R₁, R₃, R₄ and R₅ are as defined for a compound of formula (I), which is subsequently reacted with an amine of formula:
HN (R₆) R₂ (VII)
in which R₂ and R₆ are as defined for a compound of formula (I), when a compound of formula (IC) in which -X- represents a -CON(R₆)- group must be prepared;
- or with an isothiocyanate of formula R₂-N=C=S (IX) in which R₂ is as defined for a compound of formula (I), when a compound of formula (ID) in which -X- represents a -CSN(R₆)- group must be prepared.

7. Compound of formula: 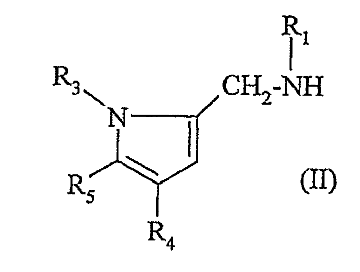 in which R₁, R₃, R₄ and R₅ are as defined for (I) Claim 1.

8. Medicament, **characterized in that** it comprises a compound of formula (I) according to any one of Claims 1 to 5, or a hydrate or a solvate of a compound of formula (I).

9. Pharmaceutical composition, **characterized in that** it comprises a compound of formula (I) according to any one of Claims 1 to 5, or a hydrate or a solvate of this compound, and also at least one pharmaceutically acceptable excipient.

10. Use of a compound of formula (I) as defined in Claims 1 to 5, for the preparation of a medicament for the treatment or prevention of diseases in which CB₁ receptors are involved.

11. Use according to Claim 10, **characterized in that** the diseases are psychiatric disorders, dependency on and withdrawal from a substance, cognitive disorders, attention and consciousness disorders, and acute and chronic neurodegenerative diseases.

12. Use according to Claim 10, **characterized in that** the diseases are metabolic disorders, appetence disorders, appetite disorders, obesity, type II diabetes, metabolic syndrome and dyslipidemia.

13. Use according to Claim 10, **characterized in that** the diseases are pain, neuropathic pain or pain induced by anticancer treatment.

14. Use according to Claim 10, **characterized in that** the diseases are gastrointestinal disorders, vomiting, diarrhea disorders, ulcers and liver diseases.

15. Use according to Claim 10, **characterized in that** the diseases are immune system diseases, rheumatoid arthritis, demyelinization, multiple sclerosis and inflammatory diseases.

16. Use according to Claim 10, **characterized in that** the diseases are Alzheimer's disease, Parkinson's disease, schizophrenia, cognitive disorders, diabetes, obesity, metabolic syndrome and tobacco withdrawal.

## Patentansprüche

1. Verbindung der Formel (I): 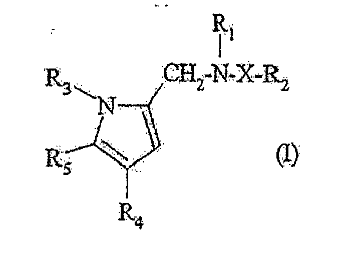 worin:
- X für eine Gruppe 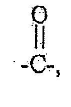 -SO₂-, 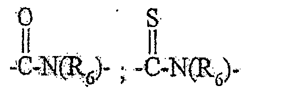 steht;
- R₁ für ein Wasserstoffatom oder eine (C₁-C₄) - Alkylgruppe steht;
- R₂ für:
. (C₁-C₁₂) -Alkyl, das gegebenenfalls ein- oder mehrfach durch unabhängig voneinander unter einem Halogenatom, Hydroxyl, (C₁-C₄) -Alkoxy, (C₁-C₄) - Alkylthio, Phenoxy, einem Trifluormethoxyrest, einem Difluormethoxyrest, einem Difluormethylthiorest und einem Trifluormethylthiorest ausgewählte Substituenten substituiert ist;
. einen nichtaromatischen carbocyclischen (C₃-C₁₂)-Rest, der gegebenenfalls ein- oder mehrfach durch unabhängig voneinander unter einem (C₁-C₄)-Alkyl, (C₁-C₄) -Alkoxy, (C₁-C₄) -Alkylthio, einem Fluoratom, Hydroxyl, einem Trifluormethylrest, einem Difluormethylrest, einem Trifluormethoxyrest, einem Difluormethoxyrest, einem Trifluormethylthiorest und einem Difluormethylthiorest ausgewählte Substituenten substituiert ist;
. Methyl, das durch einen nichtaromatischen carbocyclischen (C₃-C₁₂) -Rest, der gegebenenfalls ein- oder mehrfach durch unabhängig voneinander unter einem (C₁-C₄) -Alkyl, (C₁-C₄) -Alkoxy, (C₁-C₄) - Alkylthio, einem Fluoratom, Hydroxyl, einem Trifluormethylrest, einem Difluormethylrest, einem Trifluormethoxyrest, einem Difluormethoxyrest, einem Trifluormethylthiorest und einem Difluormethylthiorest ausgewählte Substituenten substituiert ist, substituiert ist;
. einen Phenyl-, Benzyl-, Benzhydryl- oder Benzhydrylmethylrest, in dem jede Phenylgruppe gegebenenfalls ein- oder mehrfach durch unabhängig voneinander unter einem Halogenatom, Hydroxyl, (C₁-C₄) -Alkyl, (C₁-C₄) -Alkoxy, Methylendioxy, Cyano, Nitro, Trifluormethyl, Difluormethyl, Difluormethoxy, Trifluormethoxy, Trifluormethylthio, Difluormethylthio, einer S(O)ₙ-Alk-Gruppe, einer OS(O)ₙ-Alk-Gruppe und einer (C₁-C₄) -Alkylcarbonylgruppe ausgewählte Substituenten substituiert ist;
. einen Phenylrest, der durch einen unter Pyrrolyl, Imidazolyl, Pyridyl und Pyrazolyl ausgewählten heterocyclischen Rest substituiert ist, wobei der heterocyclische Rest gegebenenfalls ein- oder mehrfach durch einen oder mehrere unabhängig voneinander unter einem Halogenatom und einer (C₁-C₄)-Alkylgruppe ausgewählte Substituenten substituiert ist;
. einen Phenylrest, der durch Phenyl oder Phenoxy substituiert ist, in dem jede Phenylgruppe gegebenenfalls ein- oder mehrfach durch unabhängig voneinander unter einem Halogenatom, Hydroxyl, (C₁-C₄) -Alkyl, (C₁-C₄) -Alkoxy, Methylendioxy, Cyano, Nitro, Trifluormethyl, Difluormethyl, Difluormethoxy, Trifluormethoxy, Trifluormethylthio, Difluormethylthio, einer S(O)ₙ-Alk-Gruppe, einer OS (O) ₙ-Alk-Gruppe und einer (C₁-C₄)-Alkylcarbonyl-gruppe ausgewählte Substituenten substituiert ist;
. 1,2,3,4-Tetrahydronaphthalin-2-yl, das gegebenenfalls ein- oder mehrfach durch (C₁-C₄) -Alkyl oder Trifluormethyl substituiert ist;
. einen Pyrrolyl-, Imidazolyl-, Pyridyl-, Pyrazolyl-, Furyl-, Thienyl-, Oxazolyl- oder Thiazolylrest, wobei die Reste gegebenenfalls durch einen oder mehrere unabhängig voneinander unter einem Halogenatom, einer (C₁-C₄)-Alkylgruppe und einer Trifluormethylgruppe ausgewählte Substituenten substituiert sind;
. Indol-2-yl oder N-Methylindol-2-yl steht;
- R₃ für (C₁-C₅) -Alkyl oder (C₃-C₇) -Cycloalkyl steht;
- R₄ für Phenyl, das gegebenenfalls ein- oder mehrfach durch unabhängig voneinander unter einem Halogenatom, (C₁-C₄) -Alkyl, (C₁-C₄) -Alkoxy, Cyano, einem Difluormethylrest, einem Trifluormethylrest, einem Difluormethoxyrest, einem Trifluormethoxyrest, einer S(O)ₙ-Alk-Gruppe und einer OS(O)ₙ-Alk-Gruppe ausgewählte Substituenten substituiert ist, steht;
- R₅ für Phenyl, das gegebenenfalls ein- oder mehrfach durch unabhängig voneinander unter einem Halogenatom, (C₁-C₄) -Alkyl, (C₁-C₄) -Alkoxy, Cyano, einem Difluormethylrest, einem Trifluormethylrest, einem Difluormethoxyrest, einem Trifluormethoxyrest, einer S(O)ₙ-Alk-Gruppe und einer OS(O)ₙ-Alk-Gruppe ausgewählte Substituenten substituiert ist, steht;
- R₆ für ein Wasserstoffatom oder (C₁-C₄) -Alkyl steht;
- n für 0, 1 oder 2 steht;
- Alk für (C₁-C₄) -Alkyl steht;
in Basen- oder Säureadditionssalzform sowie in Hydrat- oder Solvatform.

2. Verbindung der Formel (I) nach Anspruch 1, worin:
- X für eine Gruppe 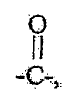 -SO₂-, 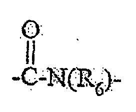 steht;
- R₁ für ein Wasserstoffatom oder eine (C₁-C₄) - Alkylgruppe steht;
- R₂ für:
. (C₁-C₇) -Alkyl;
. einen nichtaromatischen carbocyclischen C₃-C₁₂-Rest, der gegebenenfalls ein- oder mehrfach durch eine (C₁-C₄)-Alkylgruppe substituiert ist;
. Methyl, das durch einen nichtaromatischen carbocyclischen C₃-C₁₂-Rest und gegebenenfalls am Carbocyclus ein- oder mehrfach durch eine (C₁-C₄) - Alkylgruppe substituiert ist;
. Phenyl, das gegebenenfalls ein- oder mehrfach durch unabhängig voneinander unter einem Halogenatom, (C₁-C₄) -Alkyl, Hydroxyl, (C₁-C₄) - Alkoxy, Cyano, einer Trifluormethylgruppe, einer Trifluormethoxygruppe, einer S(O)ₙ-Alk-Gruppe, einer (C₁-C₄)-Alkylcarbonylgruppe oder unter einem Phenyl-, Phenoxy-, Pyrrolyl-, Imidazolyl-, Pyridyl- oder Pyrazolylrest, wobei die Reste gegebenenfalls ein- oder mehrfach durch (C₁-C₄) - Alkyl substituiert sind, ausgewählte Substituenten substituiert ist;
. Benzyl, das gegebenenfalls am Phenyl ein- oder mehrfach durch unabhängig voneinander unter einem Halogenatom, Cyano, (C₁-C₄) -Alkyl, (C₁-C₄) -Alkoxy, einem Trifluormethylrest oder einer S(O)ₙAlk-Gruppe ausgewählte Substituenten oder in alpha-Stellung durch eine oder zwei Gruppen, die gleich oder verschieden sind und unter (C₁-C₄) -Alkyl und (C₃-C₇)-Cycloalkyl ausgewählt sind, substituiert ist;
. Benzhydryl oder eine Benzhydrylmethylgruppe;
. 1,2,3,4-Tetrahydronaphthalin-2-yl, das gegebenenfalls ein- oder mehrfach durch (C₁-C₄) -Alkyl substituiert ist;
. einen Pyrrolyl-, Imidazolyl-, Pyridyl-, Pyrazolyl-, Furyl- oder Thienylrest, wobei die Reste gegebenenfalls durch einen oder mehrere unabhängig voneinander unter einem Halogenatom und einer (C₁-C₄)-Alkylgruppe ausgewählte Substituenten substituiert sind;
. Indol-2-yl oder N-Methylindol-2-yl steht;
- R₃ für (C₁-C₅) -Alkyl oder (C₃-C₇) -Cycloalkyl steht;
- R₄ für Phenyl, das gegebenenfalls ein- oder mehrfach durch unabhängig voneinander unter einem Halogenatom, (C₁-C₄)-Alkyl, (C₁-C₄) -Alkoxy, einem Trifluormethylrest oder einer S(O)ₙ-Alk-Gruppe substituiert ist, steht;
- R₅ für Phenyl, das gegebenenfalls ein- oder mehrfach durch unabhängig voneinander unter einem Halogenatom, (C₁-C₄) -Alkyl, (C₁-C₄) -Alkoxy, einem Trifluormethylrest oder einer S(O)ₙ-Alk-Gruppe ausgewählte Substituenten substituiert ist, steht;
- R₆ für ein Wasserstoffatom oder (C₁-C₄) -Alkyl steht;
- n für 0, 1 oder 2 steht;
- Alk für (C₁-C₄)-Alkyl steht;
in Basen- oder Säureadditionssalzform sowie in Hydrat- oder Solvatform.

3. Verbindung nach Anspruch 1 der Formel (IA), worin -X- für eine -CO-Gruppe steht und die Substituenten R₁ bis R₅ die für die Verbindungen der Formel (I) in Anspruch 1 angegebene Bedeutung besitzen.

4. Verbindung nach Anspruch 1 der Formel (IB), worin -X- für eine -SO₂-Gruppe steht und die Substituenten R₁ bis R₅ die für die Verbindungen der Formel (I) in Anspruch 1 angegebene Bedeutung besitzen.

5. Verbindung nach Anspruch 1 der Formel (IC), worin -X- für eine -CON(R₆)-Gruppe steht und die Substituenten R₁ bis R₆ die für die Verbindungen der Formel (I) in Anspruch 1 angegebene Bedeutung besitzen.

6. Verfahren zur Herstellung einer Verbindung der Formel (I) nach Anspruch 1, **dadurch gekennzeichnet, daß** man:
eine Verbindung der Formel: 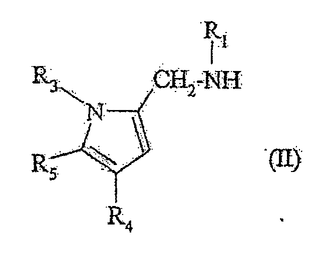 worin R₁, R₃, R₄ und R₅ die für eine Verbindung der Formel (I) angegebene Bedeutung besitzen,
- entweder mit einer Säure oder einem funktionellen Derivat dieser Säure der Formel:
HOOC-R₂ (III)
worin R₂ die für eine Verbindung der Formel (IA) angegebene Bedeutung besitzt, behandelt, wenn eine Verbindung der Formel (IA), worin -X- für eine -CO-Gruppe steht, hergestellt werden soll;
- oder mit einem Sulfonylhalogenid der Formel:
Hal-SO₂-R₂ (IV)
worin R₂ die für eine Verbindung der Formel (I) angegebene Bedeutung besitzt und Hal für ein Halogenatom, vorzugsweise Chlor, steht, behandelt, wenn eine Verbindung der Formel (IB), worin -X-für eine -SO₂-Gruppe steht, hergestellt werden soll;
- oder mit einem Halogenformiat der Formel:
HalCOOAr (V)
worin Hal für ein Halogenatom steht und Ar für Phenyl oder 4-Nitrophenyl steht, behandelt, wobei man eine Zwischenverbindung der Formel: 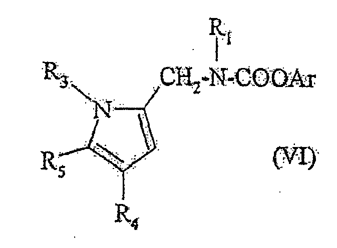 worin R₁, R₃, R₄ und R₅ die für eine Verbindung der Formel (I) angegebene Bedeutung besitzen, erhält, die man danach mit einem Amin der Formel:
HN(R₆)R₂ (VII)
worin R₂ und R₆ die für eine Verbindung der Formel (I) angegebene Bedeutung besitzen, umsetzt, wenn eine Verbindung der Formel (IC), worin -X- für eine -CON(R₆)-Gruppe steht, hergestellt werden soll;
- oder mit einem Isothiocyanat der Formel R₂-N=C=S (IX), worin R₂ die für eine Verbindung der Formel (I) angegebene Bedeutung besitzt, behandelt, wenn eine Verbindung der Formel I (D), worin -X- für eine -CSN(R₆)-Gruppe steht, hergestellt werden soll.

7. Verbindung der Formel: 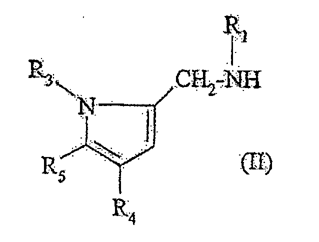 worin R₁, R₃, R₄ und R₅ die für (I) in Anspruch 1 angegebene Bedeutung besitzen.

8. Arzneimittel, **dadurch gekennzeichnet, daß** es eine Verbindung der Formel (I) nach einem der Ansprüche 1 bis 5 oder ein Hydrat oder ein Solvat einer Verbindung der Formel (I) enthält.

9. Pharmazeutische Zusammensetzung, **dadurch gekennzeichnet, daß** sie eine Verbindung der Formel (I) nach einem der Ansprüche 1 bis 5 oder ein Hydrat oder ein Solvat dieser Verbindung sowie mindestens einen pharmazeutisch unbedenklichen Hilfsstoff enthält.

10. Verwendung einer Verbindung der Formel (I) gemäß den Ansprüchen 1 bis 5 zur Herstellung eines Arzneimittels zur Behandlung oder Prävention von Erkrankungen, an denen die CB₁-Rezeptoren beteiligt sind.

11. Verwendung nach Anspruch 10, **dadurch gekennzeichnet, daß** es sich bei den Erkrankungen um psychiatrische Störungen, Substanzabhängigkeit und -entwöhnung, kognitive Störungen, Aufmerksamkeits- und Wachsamkeitsstörungen und akute und chronische neurodegenerative Erkrankungen handelt.

12. Verbindung nach Anspruch 10, **dadurch gekennzeichnet, daß** es sich bei den Erkrankungen um Stoffwechselstörungen, Appetenzstörungen, Appetitstörungen, Obesitas, Typ-II-Diabetes, metabolisches Syndrom und Dyslipidämie handelt.

13. Verwendung nach Anspruch 10, **dadurch gekennzeichnet, daß** es sich bei den Erkrankungen um Schmerzen, neuropathische Schmerzen und durch Antikrebsbehandlung induzierte Schmerzen handelt.

14. Verwendung nach Anspruch 10, **dadurch gekennzeichnet, daß** es sich bei den Erkrankungen um Magen-Darm-Störungen, Erbrechen, Durchfallstörungen, Geschwüre und Lebererkrankungen handelt.

15. Verwendung nach Anspruch 10, **dadurch gekennzeichnet, daß** es sich bei den Erkrankungen um Erkrankungen des Immunsystems, rheumatoide Arthritis, Demyelinisierung, multiple Sklerose und entzündliche Erkrankungen handelt.

16. Verwendung nach Anspruch 10, **dadurch gekennzeichnet, daß** es sich bei den Erkrankungen um Alzheimer-Krankheit, Parkinson-Krankheit, Schizophrenie, kognitive Störungen, Diabetes, Obesitas, metabolisches Syndrom und Tabakentwöhnung handelt.
